# EUROPEAN PATENT SPECIFICATION

(11) **EP 1 713 475 B1**
(45) Date of publication and mention of the grant of the patent: **30.07.2008**
(21) Application number: 05707857.8
(22) Date of filing: 27.01.2005
(51) Int. Cl.: A61K 31/4155, C07D 231/06, A61P 25/00

(54) **1,3,5-TRISUBSTITUTED 4,5-DIHYDRO-1H-PYRAZOLE DERIVATIVES HAVING CB1-ANTAGONISTIC ACTIVITY**
1,3,5-TRISUBSTITUIERTE 4,5-DIHYDRO-1H-PYRAZOL-DERIVATIVE MIT CB1-ANTAGONISTISCHER AKTIVITÄT
DERIVES DE 4,5-DIHYDRO-1H-PYRAZOLE 1,3,5-TRISUBSTITUES PRESENTANT UNE ACTIVITE ANTAGONISTIQUE A CB1

(30) Priority: 30.01.2004 US 539983 P; 30.01.2004 EP 04100338
(43) Date of publication of application: 25.10.2006
(73) Proprietor: Solvay Pharmaceuticals B.V., 1381 CP Weesp (NL)
(72) Inventor: LANGE, Josephus, H.M., NL-1381 CP Weesp (NL); KRUSE, Cornelis, G., NL-1381 CP Weesp (NL); VAN STUIVENBERG, Herman, H., NL-1381 CP Weesp (NL)
(74) Representative: Verhage, Marinus
(86) International application number: PCT/EP2005/050339
(87) International publication number: WO 2005/074920

(56) References cited:
- WO-A-01/70700
- WO-A-03/026647
- MESCHLER J P ET AL: "Inverse agonist properties of N-(piperidin-1-yl)-5-(4-chlorophenyl)- 1-(2, dichlorophenyl)-4-methyl-1H-pyrazole-3- carboxamide HCl (SR141716A) and 1-(2-chlorophenyl)-4-cyano-5-(4-methoxyphe nyl)-1H-pyr azole-3-carboxylic acid phenylamide (CP-272871) for the CB1 cannabinoid receptor" BIOCHEMICAL PHARMACOLOGY, PERGAMON, OXFORD, GB, vol. 60, 2000, pages 1315-1323, XP002262541 ISSN: 0006-2952

## Description

The present invention relates to 1,3,5-trisubstituted 4,5-dihydro-1H-pyrazole derivatives as CB₁ antagonists, to methods for the preparation of these compounds and to novel intermediates useful for the synthesis of said pyrazole derivatives. The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the Invention specific compounds disclosed herein are used for the manufacture of a medicament useful in the treatment of disorders in which cannabinoid receptors are involved, or that can be treated via manipulation of those receptors.

in WO 8805046, N-Aryl-4,5-dihydro-1H-pyrazole-3-carboxamide derivatives have been described as insecticides, but not as cannabinoid receptor antagonists. 3,4-Diaryl-4,5-dihydropyrazole-1-carboxamidine derivatives are known as CB₁ receptor antagonists (see WO 0170700, WO 0276949, WO 0326647 and WO 02664 8). However, the 4,5-dihydropyrazole derivatives described in this invention have a significantly different 1,3,5-substitution pattern and as a consequence have to be prepared via a entirely different synthetic route.

It has now surprisingly been found that potent and selective antagonism or inverse agonism of cannabinoid-CB₁, receptors is present in the novel 1,3,5-trisubstituted 4,5-dihydro-1H-pyrazole derivatives of the formula (I), tautomers thereof and salts thereof 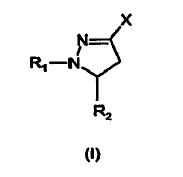 wherein:
- R₁ and R₂ independently represent phenyl, thienyl or pyridyl which groups may be substituted with 1, 2 or 3 substituents Y, which can be the same or different, from the group branched or linear C₁₋₃-alkyl or alkoxy, phenyl, hydroxy, chloro, bromo, fluoro, iodo, trifluoromethyl, trifluoromethylthio, trifluoromethoxy, methylsulfonyl, carboxyl, trifluoromethylsulfonyl, cyano, carbamoyl, sulfamoyl and acetyl, or R₁ and/or R₂ represent naphtyl,
- X represents one of the subgroups (i) or (ii), 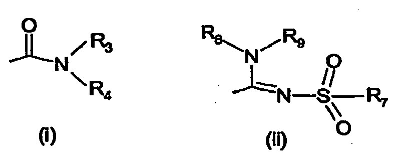 wherein
   - R₉ represents a hydrogen atom or a branched or linear C₁₋₃ alkyl group,
   - R₄ represents a branched or linear C₁₋₈ alkyl or C₃₋₈-cycloalkyl-C₁₋₂-alkyl group, branched or linear C₁₋₈ alkoxy, C₃₋₈ cycloalkyl, C₅₋₁₀ bicycloalkyl, C₆₋₁₀ bicycloalkyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, hydroxy or trifluoromethyl group or a fluoro atom, or
      R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, amino, hydroxy or trifluoromethyl group or a fluoro atom,
   - R₇ represents a benzyl, phenyl, thienyl or pyridyl group, which groups may be substituted on their aromatic ring with 1, 2, 3 or 4 substituents Y, wherein Y has the meaning as indicated above, which can be the same or different, or R₇ represents C₁₋₈ branched or linear alkyl, C₃₋₈ alkenyl, C₃₋₁₀ cycloalkyl, C₅₋₁₀ bicycloalkyl, C₆₋₁₀ tricycloalkyl or C₅₋₈ cycloalkenyl or R₇ represents naphtyl or R₇ represents an amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl, phenyl, hydroxy or trifluoromethyl group or a fluoro atom,
   - R₈ represents a hydrogen atom or a branched or linear C₁₋₃ alkyl group,
   - R₉ represents a hydrogen atom or a branched or linear C₁₋₈ alkyl, C₃₋₈ cycloalkyl or C₂₋₁₀ heteroalkyl group which groups may be substituted with a keto group, trifluoromethyl group or a fluoro atom, or R₉ represents an amino, hydroxy, phenoxy or benzyloxy group or R₉ represents a branched or linear C₁₋₈ alkoxy group, which may be substituted with a hydroxy group, a trifluoromethyl group or a fluoro atom, or R₉ represents a phenyl, benzyl, pyridyl, thienyl, pyridylmethyl or phenethyl group wherein the aromatic rings may be substituted with 1, 2 or 3 of the substituents Y, wherein Y has the meaning as indicated above, or
      R₉ represents a group NR₁₀R₁₁ with the proviso that R₈ represents a hydrogen atom or a methyl group and wherein R₁₀ and R₁₁ are the same or different and represent C₁₋₄ alkyl or C₂₋₄ trifluoroalkyl or R₁₀ and R₁₁- together with the nitrogen atom to which they are bonded - form a saturated or unsaturated heterocyclic moiety having 4 to 8 ring atoms which heterocyclic moiety contains one or two atoms from the group (O, N, S) which saturated or unsaturated heterocyclic moiety may be substituted with a C₁₋₂ alkyl group or
      R₈ and R₉ - together with the nitrogen atom to which they are bonded - form a saturated or unsaturated, monocyclic or bicyclic heterocyclic moiety having 4 to 10 ring atoms, which heterocyclic moiety contains one or two atoms from the group (O, N, S) or a keto group or -SO₂- group, which moiety may be substituted with a C₁₋₂ alkyl, hydroxy, phenyl, methylamino, dimethylamino, azetidinyl, pyrrolidinyl, piperidinyl or hexahydro-1H-azepinyl group.

At least one centre of chirality is present (at the C₅ position of the 4,5-dihydro-1H-pyrazole moiety) in the compounds of the formula (I). The invention relates both to racemates, mixtures of diastereomers and the individual stereoisomers of the compounds having formula (I). The invention also relates both to the E isomer, Z isomer and E/Z mixtures of compounds having formula (I).

Prodrugs are therapeutic agents which are inactive per se but are transformed into one or more active metabolites. Prodrugs are bioreversible derivatives of drug molecules used to overcome some barriers to the utility of the parent drug molecule. These barriers include, but are not limited to, solubility, permeability, stability, presystemic metabolism and targeting limitations (Medicinal Chemistry: Principles and Practice, 1994, ISBN 0-85186-494-5, Ed.: F. D. King, p. 215; J. Stella, "Prodrugs as therapeutics-, Expert Opin. Ther. Patents, 14(3), 277-280, 2004; P. Ettmayer et al., "Lessons learned from marketed and investigational prodrugs", J.Med.Chem., 47, 2393-2404, 2004). Pro-drugs, i.e. compounds which when administered to humans by any known route, are metabolised to compounds having formula (1), belong to the invention. In particular this relates to compounds with primary or secondary amino or hydroxy groups. Such compounds can be reacted with organic acids to yield compounds having formula (1) wherein an additional group is present which is easily removed after administration, for instance, but not limited to amidine, enamine, a Mannich base, a hydroxyl-methylene derivative, an O-(acyloxymethylene carbamate) derivative, carbamate, ester, amide or enaminone.

The invention particularly relates to compounds having formula (I) 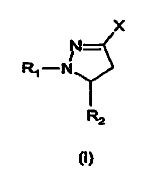 wherein
- R₁ and R₂ independently represent phenyl, which phenyl group may be substituted with 1, 2 or 3 substituents Y, which can be the same or different, from the group branched or linear C₁₋₃-alkyl or alkoxy, phenyl, hydroxy, chloro, bromo, fluoro, iodo, trifluoromethyl, trifluoromethylthio, trifluoromethoxy, methylsulfonyl, carboxyl, trifluoromethylsulfonyl, cyano, carbamoyl, sulfamoyl and acetyl, or R₁ and/or R₂ represent naphtyl, thienyl or pyridyl,
- X represents one of the subgroups (i) or (ii), 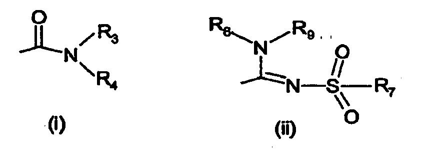 wherein
   - R₃ represents a hydrogen atom,
   - R₄ represents a branched or linear C₁₋₈ alkyl, branched or linear C₁₋₈ alkoxy or C₃₋₈ cycloalkyl group, which groups may be substituted with a hydroxy group, 1-3 methyl groups, an ethyl group or 1-3 fluoro atoms, or R₄ represents a phenoxy, pyridyl or thienyl group, or R₄ represents a group NR₅R₆ wherein
      R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O, N, S) or
      R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a methyl, hydroxy or trifluoromethyl group or a fluoro atom,
   - R₇ represents a phenyl group, which phenyl group may be substituted on its aromatic ring with 1, 2, 3 or 4 substituents Y, wherein Y has the meaning as indicated above, which can be the same or different, or R₇ represents C₁₋₈ branched or linear alkyl, C₃₋₁₀ cycloalkyl or C₅₋₁₀ bicycloalkyl, or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl or hydroxy group.
   - R₈ represent a hydrogen atom or a branched or linear C₁₋₃ alkyl group,
   - R₉ represents a hydrogen atom or a branched or linear C₁₋₈ alkyl or C₃₋₈ cycloalkyl group which groups may be substituted with a trifluoromethyl group or a fluoro atom, or R₉ represents an amino, hydroxy, phenoxy or benzyloxy group or R₉ represents a branched or linear C₁₋₈ alkoxy group, or R₉ represents a phenyl group wherein the aromatic ring may be substituted with 1, 2 or 3 of the substituents Y, wherein Y has the meaning as indicated above, or
      R₉ represents a group NR₁₀R₁₁ with the proviso that R₈ represents a hydrogen atom or a methyl group and wherein R₁₀ and R₁₁ are the same or different and represent C₁₋₄ alkyl or C₂₋₄ trifluoroalkyl or R₁₀ and R₁₁- together with the nitrogen atom to which they are bonded - form a saturated or unsaturated heterocyclic moiety having 4 to 8 ring atoms which heterocyclic moiety contains one or two atoms from the group (O, N, S) or
      R₈ and R₉- together with the nitrogen atom to which they are bonded - form a saturated or unsaturated, monocyclic or bicyclic heterocyclic moiety having 4 to 10 ring atoms, which heterocyclic moiety contains one or two atoms from the group (O, N, S) or a keto group or -SO₂- group
and tautomers, stereoisomers, prodrugs and salts thereof.

It was also found that compounds with the general formula (I) in which the meaning of R₄ is phenyl, i.e. compounds described in WO 8805046, are active as CB₁ receptor antagonists.

Due to the potent CB₁ antagonistic activity the compounds according to the invention are suitable for use in the treatment of psychiatric disorders such as psychosis, anxiety, depression, attention deficits, memory disorders, cognitive disorders, appetite disorders, obesity, in particular juvenile obesity and drug induced obesity, addiction, impulse control disorders, appetence, drug dependence and neurological disorders such as neurodegenerative disorders, dementia, dystonia, muscle spasticity, tremor, multiple sclerosis, traumatic brain injury, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuro pathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, cardiovascular disorders, atherosclerosis, liver cirrhosis and sexual disorders.

The cannabinoid receptor modulating activity of the compounds of the invention makes them particularly useful in the treatment of obesity, juvenile obesity and drug induced obesity, when used in combination with lipase inhibitors. Specific examples of compounds which can be used in such combination preparations are (but not restricted to) the synthetic lipase inhibitor orlistat, lipase inhibitors isolated from micro organisms such as lipstatin (from *Streptomyces toxytricini),* ebelactone B (from *Streptomyces aburaviensis),* synthetic derivatives of these compounds, as well as extracts of plants known to possess lipase inhibitory activity, for instance extracts of *Alpinia officinarum* or compounds isolated from such extracts like 3-methylethergalangin (from *A officinarum).*

### GENERAL ASPECTS OF SYNTHESES

The synthesis of compounds having formula (I) wherein X represents subgroup (i) is outlined in Scheme 1. intermediates having gener al formula (VII) can be obtained according to methods known, see for example: WO88/05046 and references cited therein. Intermediates having formula (V) can be obtained according to methods known, for example: Shawall et al., J. Heterocyclic Chem. 2003, 40 (2), 207 and references cited therein.

Diazotation of compounds having formula (II) can give a diazonium chloride of formula (III) by treatment with NaNO₂ under acidic conditions (HCl). Coupling of (III) with an alkyl 2-chloro-3-oxobutanoate derivative, such as ethyl 2-chloro-3-oxobutanoate (IV) can give a 2 -chloro(hydrazono)acetate derivative of general formula (V). Reaction of (V) with an alkene derivative of formula (VI) can give a 1,5-disubstituted-4,5-dihydro-1H-pyrazole-3-carboxylate analogue of general formula (VII). A compound of general formula (VII) can react with an amine R₃R₄NH, preferably in the presence of trimethylaluminum (Me₃Al) to give a compound of formula (I), wherein X represents subgroup (i) and R₃ and R₄ have the meaning as given above on page 2. More information on trimethylaluminum Al(CH₃)₃ promoted amidation reactions of esters can be found in: J. I. Levin, E. Turos, S. M. Weinreb, Synth Commun. (1982), 12, 989-993.

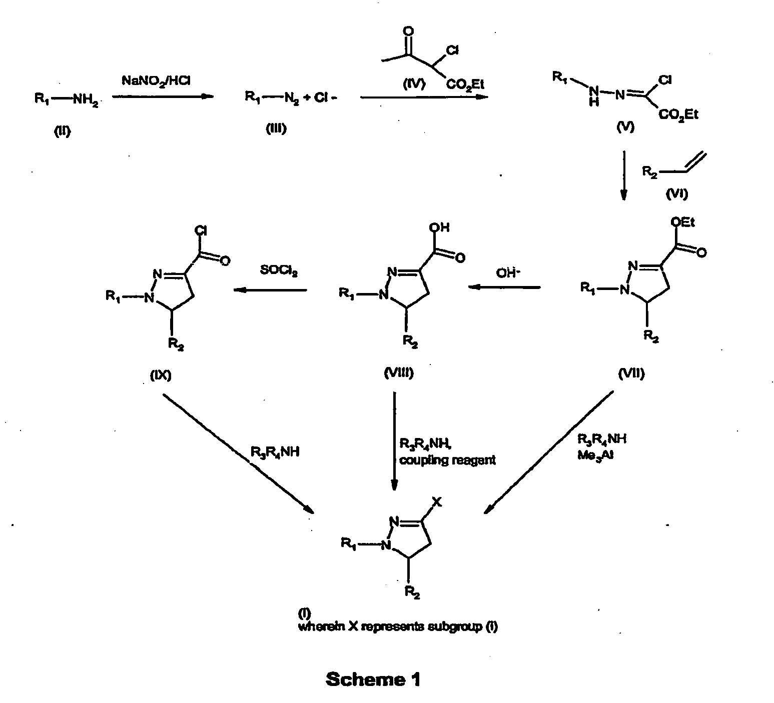

Alternatively, a compound of general formula (VII) can be hydrolysed to the corresponding carboxylic acid (VIII). The resulting carboxylic acid (VIII) can be reacted with an amine R₃R₄NH to give a compound of formula (I), wherein X represents subgroup (i) and R₃ and R₄ have the meaning as given above on page 2, *via* activating and coupling methods such as formation of an active ester, or in the presence of a so-called coupling reagent, such as for example, DCC, HBTU, BOP, CIP (2-chloro-1,3-dimethylimidazolinium hexafluorophos-phate), PyAOP (7-azabenzotriazol-1-yloxytris(pyrrolidino) phos-phonium hexa-fluorophosphate) and the like. More information on activating and coupling methods of amines to carboxylic acids can be found in:
a) M. Bodanszky and A. Bodanszky: The Practice of Peptide Synthesis, Springer-Veriag, New York, 1994; ISBN: 0-387-57505-7;
b) K. Akaji et al., Tetrahedron Lett. (1994),35,3315-3318);
c) F. Albericio et al., Tetrahedron Lett. (1997), 38,4853-4856).

Alternatively, carboxylic acid (VIII) can be reacted with a halogenating agent such as thionyl chloride (SOCl₂) to give the corresponding carbonyl chloride (IX). Compound (IX) can be reacted with an amine R₃R₄NH to give a compound of formula (I), wherein X represents subgroup (i) and R₁, R₂, R₃ and R₄ have the meaning as given above on page 2.

The synthesis of compounds having formula (i) wherein X represents subgroup (ii) is outlined in Scheme 2.

A compound of general formula (VII) can be converted to the corresponding carboxamidine derivative (X) for example by using trimethylaluminum (Me₃Al) and NH₄Cl, followed by a treatment with aqueous base. Information on such a conversion of an ester into a carboxamidine can be found in Tetrahedron. Lett. 2002, 43, 419 (Gielen et al.). Compounds of general formula (X) wherein R₁ and R₂ have the meaning as given above on page 2 are new. A compound of general formula (X) can be reacted with a sulfonyl chloride R₇SO₂Cl in the presence of a base such as N-diisopropylethylamine (DIPEA) to give a compound of formula (I), wherein X represents subgroup (ii) and R_{1,} R₂, and R₇ have the meaning as given above on page 2, and R₈ and R₉ represent a hydrogen atom.

An intermediate of general formula R₇SO₂NH₂ can be prepared from the corresponding compound R₇SO₂Cl or by synthetically related protocols (see for example; McManus et al., J. Med. Chem. 1965, 8, 766). A carbonyl chloride of general formula (IX) can be reacted with a compound of general formula R₇SO₂NH₂ in the presence of a base such as for example NaH to give a compo und of general formula (XI) wherein R₁, R₂, and R₇ have the meaning as given above on pages 2 and 3. Compounds of general formula (XI) wherein R₁, R₂ and R₇ have the meaning as given above on pages 2 and 3 are new. The compound of formula (XI) can be reacted with a halogenating agent, for example a chlorinating agent such as PCl₅ and the like to give a compound of formula (XII), wherein Z represents a chloro or bromo atom. Compounds of general formula (XII) wherein R₁, R₂ and R₇ have the meaning as given above on page 2 are new. Compound (XII) can be reacted with an amine of general formula R₈R₉NH to give a compound of general formula (I), wherein X represents subgroup (ii) and R_{1,} R₂, R₇, R₈ and R₉ have the meaning as given above on the pages 1-3.

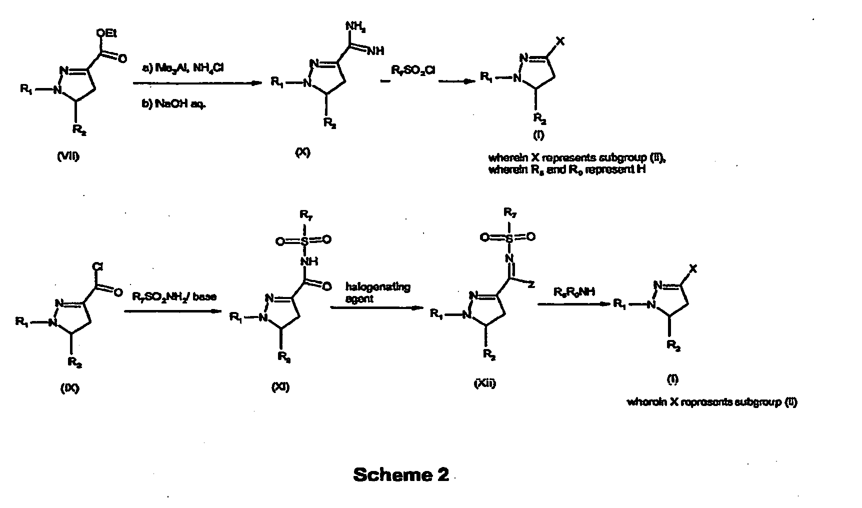

The selection of the particular synthetic method depends on factors such as the compatibility of functional groups with the reagents used, the possibility to use protecting groups, catalysts, activating and coupling reagents and the ultimate structural features present in the final compound being prepared.

According to these procedures the following compounds can be prepared. They are intended to further illustrate the invention in more detail, and therefore are not deemed to restrict the scope of the invention in any way.

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer su bstances. Frequently used auxiliary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### In vitro affinity for cannabinoid-CB₁ receptors

The affinity of the compounds of the invention for cannabinoid CB₁ receptors can be determined using membrane preparations of Chinese hamster ovary (CHO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro cannabinoid-CB₁ receptor antagonism

In vitro CB₁ receptor antagonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [³H]-arachidonic acid and incubated overnight in a cell culture stove (5% CO₂/95% air, 37°C; water-saturated atmosphere). During this period [³H]-arachidonic acid is incorporated in membrane phospholipids. On the test day, medium is aspirated and cells are washed three times using 0.5 ml DMEM, containing 0.2% bovine serum albumin (BSA). Stimulation of the CB₁ receptor by WIN 55,212-2 leads to activation of PLA₂ followed by release of [³H]-arachidonic add into the medium. This WIN 55,212-2-induced release is concentration -dependently antagonized by CB₁ receptor antagonists.

### In vivo cannabinoid-CB₁ receptor antagonism

In vivo CB₁ antagonism can be assessed with the CP-55,940-induced hypotension test in rat. Male normotensive rats (225-300 g; Harlan, Horst, The Netherlands) are anaesthetized with pentobarbital (80 mg/kg ip). Blood pressure is measured, via a cannula inserted into the left carotid artery, by means of a Spectramed DTX -plus pressure transducer (Spectramed B.V., Bilthoven, The Netherlands). After amplification by a Nihon Kohden Carrier Amplifier (Type AP-621G; Nihon Kohden B.V., Amsterdam, The Netherlands), the blood pressure signal is registered on a personal computer (Compaq Deskpro 386s), by means of a Po-Ne-Mah data-acquisition program (Po-Ne-Mah Inc., Storrs, USA). Heart rate is derived from the pulsatile pressure signal. All compounds are administered orally as a microsuspension in 1% methylcellulose 30 minutes before induction of the anesthesia which is 60 minutes prior to administration of the CB₁ receptor agonist CP-55,940. The injection volume is 10 ml/kg. After haemodynamic stabilization the CB₁ receptor agonist CP-55,940 (0.1 mg/kg *i.v.)* is administered and the hypotensive effect established. (Wagner. J. A.; Jaral, Z.; Batkai, S.; Kunos, G. Hemodynamic effects of cannabinoids: coronary and cerebral vasodilation mediated by cannabinoid CB1 receptors. Eur.J.Pharmacol. 2001, 423, 203-10).

Pharmaceutically acceptable salts may be obtained using standard procedures well known in the art, for example by mixing a compound of the present invention with a suitable acid, for instance an inorganic add such as hydrochloric acid, or with an organic acid.

### DOSE

The affinity of the compounds of the invention for cannabinoid receptors was determined as described above. From the binding affinity measured for a given compound of formula (1), one can estimate a theoretical lowest effective dose. At a concentration of the compound equal to twice the measured Kᵢ-value, 100% of the cannabinoid receptors likely will be occupied by the compound. Converting that concentration to mg of compound per kg of patient yields a theoretical lowest effective dose, assuming ideal bioavailability. Pharmaco-kinetic, pharmacodynamic, and other considerations may alter the dose actually administered to a higher or lower value. The dosage expediently administered is 0.001 - 1000 mg/kg. preferably 0.1-100 mg/kg of patient's bodyweight.

### EXAMPLES

### EXAMPLE 1: SYNTHESES OF SPECIFIC COMPOUNDS

### Compounds 1 -4

Part A: To a stirred solution of 4-chloroaniline (15.68 gram, 0.123 mol) in ice (30 ml) and concentrated hydrochloric acid (30 ml) is slowly added a solution of NaNO₂ (9.0 gram, 0.13 mol) in water (16 ml) and the resulting solution is stirred for 1 hour at 0-5 °C and subsequently added to a cold mixture of NaOAc (32 gram, 0.39 mol), ethanol (520 ml) and ethyl 2-chloro-3-oxobutanoate (16.6 ml, 0.12 mol). After stirring the resulting mixture for 1 hour the formed precipitate is collected by filtration, washed with ethanol and dried in vacuo to give ethyl 2-chloro[(4-chlorophenyl)hydrazono]acetate (22.99 gram, 73 % yield). Melting point: 147.5-149.5 °C. ¹H-NMR (200 MHz, CDCl₃): δ 1.40 (t, J = 7 Hz, 3H), 4.39 (q, J = 7 Hz, 2H), 7.16 (br d, J = 8 Hz, 2H), 7.30 (br d, J = 8 Hz, 2H), 8.31 (br s, 1H).

Part B: To a stirred boiling solution of ethyl 2-chloro[(4-chlorophenyl) hydrazono]acetate (22.95 gram, 0.088 mol) and styrene (30.3 ml, 0.264 mol) in benzene (140ml) is added triethylamine (34.3 ml, 0.247 mol)and the resulting solution is heated at reflux temperature for 1 hour. The resulting solution is cooled to room temperature and the formed precipitate is removed by filtration and washed with toluene. The filtrate is concentrated *in vacuo* and purified by flash chromatography (silica gel, dichloromethane) to give ethyl 1-(4-chlorophenyl)-5-phenyl-4,5-dihydro-(1H)-pyrazole-3-carboxylate (272 gram, 94% yield) as a syrup which slowly solidifies on standing. ¹H-NMR (200 MHz, CDCl₃): δ 1.38 (t, J = 7 Hz, 3H), 3.06 (dd, J = 18 and 7 Hz, 1H), 3.73 (dd, J = 18 and 13 Hz, 1H), 4.33 (q, J = 7 Hz, 2H), 5.38 (dd, J = 13 and 7 Hz, 1H), 7.02 (br d, J = 8 Hz, 2H), 7.08-7.40 (m, 7H).

Part C: To a stirred suspension of ethyl 1-(4-chlorophenyl)-5-phenyl-4,5-dihydro-(1H)-pyrazole-3-carboxylate (23.0 gram, 0.07 mol) in methanol (200 ml) is added water (15 ml) and concentrated NaOH (10 ml) and the resulting solution is heated at reflux temperature for 2 hours. The methanol is partly removed by evaporation and the residue is dissolved in a mixture of water and ethyl acetate. Ice, concentrated HCl (20 ml) and ethyl acetate are successively added, the ethyl acetate layer collected, dried over MgSO₄, filtered and concentrated in vacuo. The resulting residue is washed with diethyl ether (100 ml) and diisopropyl ether respectively, to give 1-(4-chlorophenyl)-5-phenyl-4,5-dihydro(1H)pyrazole-3-carboxylic acid (16.46 gram, 78 % yield). Melting point 177-179 °C.

Part D: To a stirred suspension of 1-(4-chlorophenyl)-phenyl-4,5-dihydro-(1H)-pyrazole-3-carboxylic acid (1.50 gram, 5 mmol) in anhydrous acetonitrile (40 ml) is successively added N-diisopropylethylamine (DIPEA) (1.92 ml, 11 mmol), O-benzotriazol-1-yl-N, N, N', N'-tetramethyluronium hexafluorophos-phate (HBTU) (2.08 g, 5.5 mmol) and 1-aminopiperidine (0.59 ml, 5.5 mmol) and the resulting mixture is reacted at room temperature for 16 hours in a N₂ atmosphere. The mixture is concentrated and added to a mixture of ethyl acetate and aqueous NaHCO₃. The Ethyl acetate layer is collected, dried over MgSO₄, filtered and concentrated In vacuo. The resulting residue is recrystallised from acetonitrile to give N-(piperidin-1-yl)-1-(4-chlorophenyl)-5-phenyl-4,5-dihydro-(1H)-pyrazole-3-carboxamide

### (compound 1), 1.34 gram, 70 % yield). Melting point 189-192 °C.

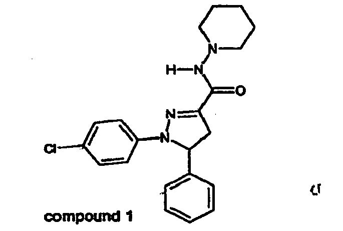

In an analogous manner the compounds having formula (I) listed below have been prepared, the compounds 2, 3 and 4:

### Compound 2: N-(Piperidin-1-yl)-5-(4-chlorophenyl)-1-(2,4-dichlorophenyl)-4,5-dihydro-(1H)-pyrazole-3-carboxamide. Melting point: 185-187 °C.

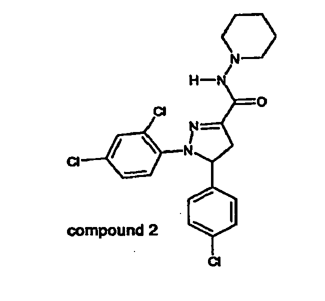

### Compound 3: N-(Piperidin-1-y)-1-(2,4-dichlorophenyl)-5-phenyl-4,5-dihydro-(1H)-pyrazole-3-carboxamide. Melting point 163-165 °C.

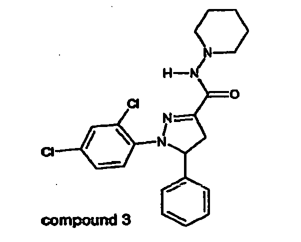

### Compound 4: N-Cyclohexyl)-1-(2,4-dichlorophenyl)-5-phenyl-4,5-dihydro-(1H)-pyrazole-3-carboxamide. Melting point: 160-163.5 °C.

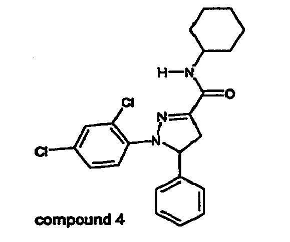

### Compounds 5 and 6

Part A: A stirred suspension of NH₄Cl (2.68g, 0.05 mol) in toluene (25 ml) is cooled to 0 °C in an N₂ atmosphere. A solution of Me₃Al in toluene (25 ml of a 2 M solution) is slowly added and the mixture is allowed to attain room temperature. A solution of ethyl 1-(4-chlorophenyl)-5-phenyl-4,5-dihydro-(1H)-pyrazole-3-carboxylate (3.29 gram, 10 mmol) in toluene (25 ml) is slowly added and the reaction mixture is stirred at 80°C for 16 hours. After cooling to 0 °C methanol is slowly added and the formed precipitate is removed by filtration. The filtrate is concentrated and the residue is dissolved in a mixture of dichloromethane and methanol. The formed precipitate is removed by filtration. The filtrate is concentrated and the remaining residue crystallised from dichloromethane to give 1-(4-chlorophenyl)-5-phenyl-4,5-dihydro-(1H)-pyrazole-3-carboxamidine.HCl (2.70 g, 73 % yield). ¹H-NMR (200 MHz, DMSO-d₆): δ 3.08 (dd, J = 18 and 7 Hz, 1H), 3:82 (dd, J = 18 and 13 Hz. 1H). 5.84 (dd, J = 13 and 7 Hz, 1H), 7.15-7.46 (m, 9H), 8.85 (br s, 2H), 9.00 (br s, 2H).
Analogously was prepared 1-(2,4-dichlorophenyl)-5-phenyl-4,5-dihydro-(1H)-pyrazole-3-carboxamidine.HCl. ¹H-NMR (200 MHz, DMSO-d₆ with addition of some trifluoroacetic acid): δ 3.32 (dd, J = 18 and 7 Hz, 1H), 3.82 (dd, J = 18 and 13 Hz, 1H), 6.08 (dd, J = 13 and 7 Hz, 1H), 7.13-7.31 (m, 6H), 7.42 (d, J = 2 Hz, 1H), 7.52 (d, J = 8 Hz, 1H), 8.95 (br s, 2H), 9.05 (br s, 2H).

Part B: To a stirred solution of 1-(4-chlorophenyl)-5-phenyl-4,5-dihydro-(1H)-pyrazole-3-carboxamidine.HCl (1.87 gram, 5 mmol) and 4-fluorophenylsulfonyl chloride (0.97 g, 5 mmol) in anhydrous acetonitrile (20 ml) is added triethylamine (2.1 ml, 15 mmol) and the resulting mixture is stirred at room temperature for 16 hours. After concentration *in vacuo,* the residue is dissolved In a mixture of Ethyl acetate and water. The Ethyl acetate layer is collected, dried over MgSO₄, filtered and concentrated in vacuo. The resulting residue is recrystallised from methyl-t-butyl ether to give N-[(4-fluorophenyl)sulfonyl]-1-(4-chlorophenyl)-5-phenyl-4,5-dihydro-(1H)-pyrazole-3-carboxamidine (1.22 gram, 54 % yield). Melting point 200-203.5 °C.

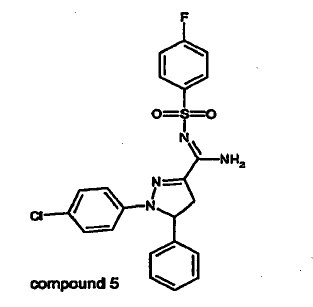

In an analogous manner compound 6 was prepared:

### Compound 6: N-[(4-fluorophenyl)sulfonyl]-1-(2.4-dichlorophenyl)-5-phenyl-4,5-dihydro-(1H)-pyrazole-3-carboxamidine. Melting point 167-169.5 °C.

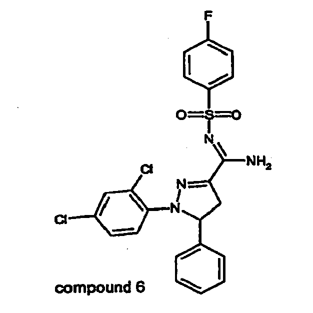

### Compounds 7 - 9

**Part A:** To a stirred solution of 1-(4-chlorophenyl)-5-phonyl-4,5-dihydro-(1H)-pyrazole-3-carboxylic acid (3.01 gram, 10 mmol) in toluene (30 ml) is added thionyl chloride (SOCl₂) (2.9 ml, 40 mmol) and the resulting mixture is heated at 80 °C for 1 hour. After thorough concentration *in vacuo* the residue is dissolved in anhydrous acetonitrile (50 ml) to give solution A. To a stirred solution 4-chlorophenylsulfonamide (1.92 gram, 10 mmol) in acetonitrile (50 ml) is added concentrated NaOH (1.3 ml, 25 mmoi). To the resulting mixture is slowly added solution A. The resulting mixture is stirred at room temperature for 16 hours. Hydrochloric acid (50 ml of a 1N solution) and water (50 ml) are added. Th e precipitate was collected by filtration, washed with water, dissolved in dichloromethane, dried over MgSO₄, filtered and concentrated in vacuo. The resulting residue is recrystallised from ethanol to give N-[(4-chlorophenyl)sulfonyl]-1-(4-chlorophenyl)-5-phenyl-4,5-dihydro-(1H)-pyrazole-3-carboxamide (4.25 gram, 79% yield). Melting point 226-229°C. Analogously were prepared N-[(4-chlorophenyl)sulfonyl]-1-(2,4-dichlorophenyl)-5-phenyl-4,5-dihydro-(1H)-pyrozole-3-carboxamide (melting point 178-181°C) and N-{[4-(trifluoromethyl)phenyl]sulfonyl}-1-(2,4-dichlorophenyl)-5-phenyl-4,5-dihydro-(1H)-pyrazole-3-carboxamide (melting point 175-177 °C).

Part B: A stirred mixture of N-[(4-chlorophenyl)sulfonyl]-1-(4-chlorophenyl)-5-phenyl-4,5-dihydro-(1H)-pyrazole-3-carboxamide (2.36 gram, 5 mmol), PCl₅ (1.15 g, 5.5 mmol) and chlorobenzene (50 ml) is heated for 90 minutes at 140 °C. After cooling the mixtuyre to room temperature the residue is dissolved in dichloromethane and methylamine.HCl (0.34 g, 5 mmol) and DIPEA (1.74 ml, 10 mmol) are successively added. The resulting mixture is stirred for 16 hours at room temperature, twice washed with water, dried over MgSO₄, filtered and concentrated in vacuo. The resulting residue is further purified by flash chromatography (silica gel, dichloromethane/acetone = 99/1 (v/v/), followed by crystallization from diisopropyl ether to give N-[(4-chlorophenyl)sulfonyl]-N'-methyl-1-(4-chlorophenyl)-5-phenyl-4,5-dihydro-(1H)-pyrazole-3-carboxamidine (0.36 gram, 15 % yield). Melting point 134-144 °C. R_{f} (silica gel, diethyl ether = 0.4).

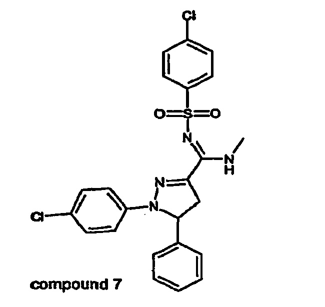

In an analogous manner the compounds having formula (I) listed below have been prepared:

### Compound 8: N-[(4-Chlorophenyl)sulfony]-N'-methyl-1-(2,4-dichlorophenyl)-5-phenyl-4,5-dihydro-(1H)-pyrazole-3-carboxamidine. Melting point 128-130.5 °C. R_{f} (silica gel, diethyl ether = 0.4).

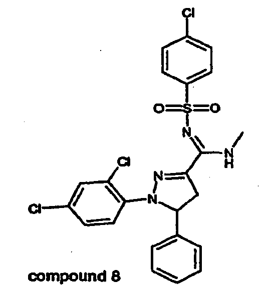

### Compound 9: N-{[(4-Trifluoromethyl)phenyl]sulfonyl}-N'-methyl-1-(2,4-dichlorophenyl)-5-phenyl-4,5-dihydro-(1H)-pyrazole-3-carboxamidine. Melting point 157-159 °C. R_{f} (silica gel, diethyl ether = 0.5).

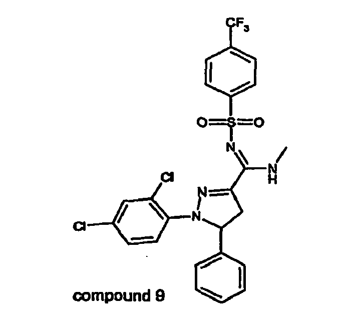

### Compounds 10 and 11

Part A: To a stirred solution of 1-(4-chlorophenyl)-5-phenyl-4,5-dihydro-(1H)-pyrazole-3-carboxylic add (3.01 gram, 10 mmol) in toluene (30 ml) is added thionyl chloride (SOCl₂) (2.9 ml, 40 mmol) and the resulting mixture is heated at 80 °C for 1 hour. After thorough concentration *in vacuo* the residue is dissolved in anhydrous acetonitrile (50 ml) to give solution B. To a ice-cooled stirred solution of pipendine-1-suffonamide (3.28 gram, 20 mmol) in anhydrous acetonitrile (50 ml) is added NaH (60 % dispersion, 0.80 g, 20 mmol) and the resulting mixture is stirred at room temperature for 1 hour. To the resulting suspension is slowly added solution B. The resulting mixture is stirred at room temperature for 16 hours and subsequently concentrated in vacuo. Hydrochloric acid (1N solution) and dichloromethane are added to the residue. The dichloromethane layer is collected, washed with water, dried over MgSO₄, filtered and concentrated in vacuo. The resulting residue is recrystallised from ethanol to give N-[(piperidin-1-yl)sulfonyl]-1-(4-chlorophenyl)-5-phenyl-4,5-dihydro-(1H)-pyrazole-3-carboxamide (3.68 gram, 82 % yield). Melting point 239.5-241.5 °C.
Analogously was prepared N-[(morpholin-4-yl)sulfonyl]-1-(2,4-dichlorophenyl)-5-phenyl-4,5-dihydro-(1H)-pyrazole-3-carboxamide. Melting point 176-179 °C.

Part B: A stirred mixture of N-[(piperidin-1-yl)sulfonyl]-1-(4-chlorophenyl)-5-phenyl-4,5-dihydro-(1H)-pyrazole-3-carboxamide (2.23 gram , 5 mmol), PCl₅ (1.15 g, 5.5 mmol) and chlorobenzene (50 ml) is heated for 90 minutes at 140 °C. After cooling the mixture to room temperature, followed by concentration in vacuo, the residue is dissolved in dichloromethane and methylamine.HCl (0.34 g, 5 mmol) and DIPEA (1.74 ml, 10 mmol) are successively added. The resulting mixture is stirred for 1 hour at room temperature, twice washed with water, dried over MgSO₄, filtered and concentrated in vacuo. The resulting residue is further purified by flash chromatography (silica gel, dichloromethane), followed by crystallization from diisopropyl ether to give N-[(piperidin-1-yl)sulfonyl]-N'-methyl-1-(4-chlorophenyl)-5-phenyl-4,5-dihydro-(1H)-pyrazole-3-carboxamidine (1.12 gram, 49 % yield). Melting point 141-144 °C. R_{f} (silica gel, diethyl ether = 0.2).

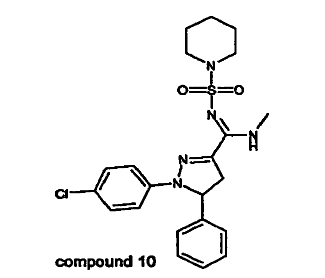

In an analogous manner the compounds having formula (I) listed below have been prepared:

### Compound 11: N-[(Morpholin-4-yl)sulfonyl]-N'-methyl-1-(2,4-dichlorophenyl)-5-phenyl-4,5-dihydro-(1H)-pyrazole-3-carboxamidine. Melting point 161-165 °C. R_{f} (silica gel, dichloramethane/acetone = 98/2 (v/v) = 0.15).

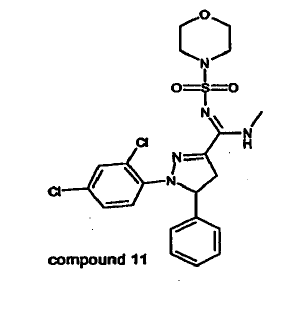

### EXAMPLE 2: FORMULATIONS USED IN ANIMAL STUDIES

**For oral *(p.o.)* administration** : to the desired quantity (0.5-6 mg) of the solid compound 3 In a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1 N). Remaining particles in the suspension were further suspended by using an ultrasonic bath.

**For intraperitoneal (*i.p*.) administration:** to the desired quantity (0.5-15 mg) of the solid compound 3 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 3: PHARMACOLOGICAL TESTRESULTS

Cannabinoid receptor affinity and functional *in vitro* data data obtained according to the protocols given above are shown in the table below.

**Table 1: pharmacological data**

| | **Human cannabinoid-CB₁ receptor** | |
|---|---|---|
| | ***In vitro* affinity** | ***In vitro* antagonism** |
| **Compound nr** | **pKᵢ value** | **% Inhibition at 10⁻⁶M** |
| | | |
| Compound 3 | **7.6** | 98% |
| Compound 10 | **7.1** | 79% |

## Claims

1. Compounds of the general formula (I) 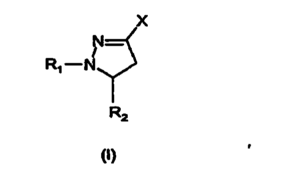 wherein:
- R₁ and R₂ independently represent phenyl, thienyl or pyridyl which groups may be substituted with 1, 2 or 3 substituents Y, which can be the same or different, from the group branched or linear C₁₋₃-alkyl or alkoxy, phenyl, hydroxy, chloro, bromo, fluoro, iodo, trifluoromethyl, trifluoromethylthio, trifluoromethoxy, methylsulfonyl, carboxyl, trifluoromethylsulfonyl, cyano, carbamoyl, sulfamoyl and acetyl, or R₁ and/or R₂ represent naphtyl,
- X represents one of the subgroups (i) or (ii), 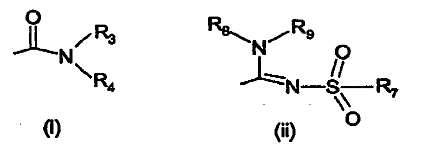 wherein:
- R₃ represents a hydrogen atom or a branched or linear C₁₋₃ alkyl group,
- R₄ represents a branched or linear C₁₋₈ alkyl or C₃₋₈-cycloalkyl-C₁₋₂-alkyl group, branched or linear C₁₋₈alkoxy. C₃₋₈ cycloalkyl, C₅₋₁₀ bicycloalkyl, C₆₋₁₀ tricycloalkyl, which groups may contain one or more heteroatoms from the group (O, N, S) and which groups may be substituted with a hydroxy group, 1-3 methyl groups, an ethyl group or 1-3 fluoro atoms, or R₄ represents a phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein
R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, hydroxy or trifluoromethyl group or a fluoro atom, or
R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, amino, hydroxy or trifluoromethyl group or a fluoro atom,
- R₇ represents a benzyl, phenyl, thienyl or pyridyl group, which groups may be substituted on their aromatic ring with 1, 2, 3 or 4 substituents Y, wherein Y has the meaning as indicated above, which can be the same or different, or R₇ represents C₁₋₈ branched or linear alkyl, C₃₋₈ alkenyl, C₃₋₁₀ cycloalkyl, C₅₋₁₀ bicycloalkyl, C₆₋₁₀ tricycloalkyl or C₅₋₈ cycloalkenyl or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl, phenyl, hydroxy or trifluoromethyl group or a fluoro atom,
- R₈ represent a hydrogen atom or a branched or linear C₁₋₃ alkyl group,
- R₉ represents a hydrogen atom or a branched or linear C₁₋₈ alkyl, C₃₋₈ cycloalkyl or C₂₋₁₀ heteroalkyl group which groups may be substituted with a keto group, trifluoromethyl group or a fluoro atom, or R₉ represents an amino, hydroxy, phenoxy or benzyloxy group or R₉ represents a branched or linear C₁₋₈ alkoxy group, which may be substituted with a hydroxy group, a trifluoromethyl group or a fluoro atom, or R₉ represents a phenyl, benzyl, pyridyl, thienyl, pyridylmethyl or phenethyl group wherein the aromatic rings may be substituted with 1, 2 or 3 of the substituents Y, wherein Y has the meaning as indicated above, or
R₉ represents a group NR₁₀R₁₁ with the proviso that R₈ represents a hydrogen atom or a methyl group and wherein R₁₀ and R₁₁ are the same or different and represent C₁₋₄ alkyl or C₂₋₄ trifluoroalkyl or R₁₀ and R₁₁- together with the nitrogen atom to which they are bonded - form a saturated or unsaturated heterocyclic moiety having 4 to 8 ring atoms which heterocyclic moiety contains one or two atoms from the group (O, N, S) which saturated or unsaturated heterocyclic moiety may be substituted with a C₁₋₂ alkyl group or
R₈ and R₉ - together with the nitrogen atom to which they are bonded - form a saturated or unsaturated, monocyclic or bicyclic heterocyclic moiety having 4 to 10 ring atoms, which heterocyclic moiety contains one or two atoms from the group (O, N, S) or a keto group or -SO₂- group, which moiety may be substituted with a C₁₋₂ alkyl, hydroxy, phenyl, methylamino, dimethylamino, azetidinyl, pyrroridinyl, piperidinyl or hexahydro-1H-azepinyl group,
and all stereoisomers, as well as salts and prodrugs, which are derivatives of the compounds having formula (I) wherein a group is present which is easily removed after administration, selected from the group consisting of an amidine, enamine, a Mannich base, a hydroxyl-methylene derivative, an O-(acyloxymethylene carbamate) derivative, carbamate or enami none.

2. Compounds as claimed in claim 1 of the general formula (I) 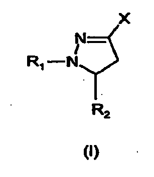 wherein:
- R₁ and R₂ independently represent phenyl, which phenyl group may be substituted with 1, 2 or 3 substituents Y, which can be the same or different, from the group branched or linear C₁₋₃-alkyl or alkoxy, phenyl, hydroxy, chloro, bromo, fluoro, iodo, trifluoromethyl, trifluoromethylthio, trifluoromethoxy, methylsulfonyl, carboxyl, trifluoromethylsulfonyl, cyano, carbamoyl, sulfamoyl and acetyl, or R₁ and/or R₂ represent naphtyl, thienyl or pyridyl,
- X represents one of the subgroups (i) or (ii), 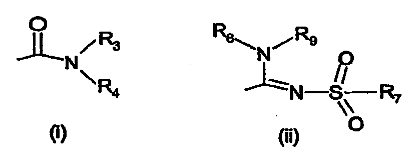 wherein:
- R₃ represents a hydrogen atom,
- R₄ represents a branched or linear C₁₋₈ alkyl, branched or linear C₁₋₈ alkoxy or C₂₋₈ cycloalkyl group, which groups may be substituted with a hydroxy group, 1-3 methyl groups, an ethyl group or 1-3 fluoro atoms, or R₄ represents a phenoxy, pyridyl or thienyl group, or R₄ represents a group NR₅R₆ wherein
R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O, N, S) or
R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a methyl, hydroxy or trifluoromethyl group or a fluoro atom,
- R₇ represents a phenyl group, which phenyl group may be substituted on its aromatic ring with 1, 2, 3 or 4 substituents Y, wherein Y has the meaning as indicated above, which can be the same or different, or R₇ represents C₁₋₈ branched or linear alkyl, C₃₋₁₀ cycloalkyl or C₅₋₁₀ bicycloalkyl, or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl or hydroxy group,
- R₈ represent a hydrogen atom or a branched or linear C₁₋₃ alkyl group,
- R₉ represents a hydrogen atom or a branched or linear C₁₋₈ alkyl or
C₃₋₈ cycloalkyl group which groups may be substituted with a trifluoromethyl group or a fluoro atom, or R₉ represents an amino, hydroxy, phenoxy or benzyloxy group or R₉ represents a branched or linear C₁₋₈alkoxy group, or R₉ represents a phenyl group wherein the aromatic ring may be substituted with 1, 2 or 3 of the substituents Y, wherein Y has the meaning as indicated above, or
R₉ represents a group NR₁₀R₁₁ with the proviso that R₈ represents a hydrogen atom or a methyl group and wherein R₁₀ and R₁₁ are the same or different and represent C₁₋₄ alkyl or C₂₋₄ trifluoroalkyl or R₁₀ and R₁₁ - together with the nitrogen atom to which they are bonded - form a saturated or unsaturated heterocyclic moiety having 4 to 8 ring atoms which heterocyclic moiety contains one or two atoms from the group (O, N, S) or
R₈ and R₉ - together with the nitrogen atom to which they are bonded - form a saturated or unsaturated, monocyclic or bicyclic heterocyclic moiety having 4 to 10 ring atoms, which heterocyclic moiety contains one or two atoms from the group (O, N, S) or a keto group or -SO₂- group
and all stereoisomers, as well as salts and prodrugs, which are derivatives of the compounds having formula (I) wherein a group is present which is easily removed after administration, selected from the group consisting of an amidine, enamine, a Mannich base, a hydroxyl-methylene derivative, an O-(acyloxymethylene carbamate) derivative, carbamate or enaminone.

3. Pharmaceutical compositions comprising, in addition to a pharmaceutically acceptable carrier and/or at least one pharmaceutically acceptable auxiliary substance, a pharmacologically active amount of at least one compound of one of the claims 1-2, or a salt thereof, as an active ingredient

4. Compounds of the general formula (X), tautomers thereof and salts thereof 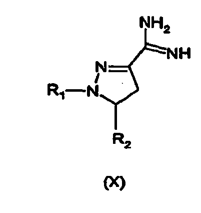 wherein R₁ and R₂ have the meanings given in claim 1, such compounds being useful in the synthesis of compounds of the general formula (I).

5. Compounds of the general formula (XI) 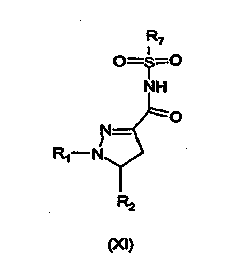 wherein R₁, R₂ and R₇ have the meanings given in claim 1, such compounds being useful in the synthesis of compounds of the general formula (I).

6. Compounds of the general formula (XII) 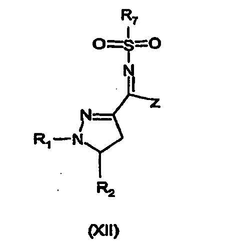 wherein R₁, R₂ and R₇ have the meanings given in claim 1 and wherein Z represents a Cl or Br atom, such compounds being useful in the synthesis of compounds of the general formula (I).

7. A compound as claimed in claim 1 or claim 2, or a salt thereof, for use in medicine.

8. Use of a compound as claimed in claim 1 or claim 2 for the preparation of a pharmaceutical composition for the treatment of disorders involving cannabinoid neurotransmission, such as psychosis, anxiety, depression, attention deficits, memory disorders, cognitive disorders, appetite disorders, obesity, in particular juvenile obesity and drug induced obesity, addiction, impulse control disorders, appetence, drug dependence and neurological disorders such as neurodegenerative disorders, dementia, dystonia, muscle spasticity, tremor, multiple sclerosis, traumatic brain injury, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, cardiovascular disorders, atherosclerosis, liver cirrhosis and sexual disorders.

9. A method of preparing a pharmaceutical composition
**characterized** i**n that** a compound of formula (I) is used 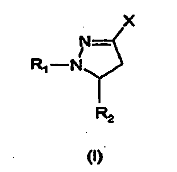 wherein:
- R₁ and R₂ independently represent phenyl, thienyl or pyridyl which groups may be substituted with 1, 2 or 3 substituents Y, which can be the same or different, from the group C₁₋₃alkyl or alkoxy, phenyl, hydroxy, chloro, bromo, fluoro, iodo, trifluoromethyl, trifluoromethylthio, trifluoromethoxy, methylsulfonyl, carboxyl, trifluoromethylsulfonyl, cyano, carbamoyl, sulfamoyl and acetyl, or R₁ and/or R₂ represent naphtyl,
- X represents one of the subgroups (i) or (ii), 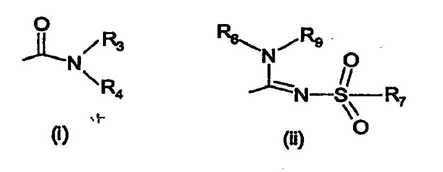
wherein:
- R₃ represents a hydrogen atom or a branched or linear C₁₋₃ alkyl group,
- R₄ represents a branched or linear C₁₋₈ alkyl or C₃₋₈-cycloalkyl-C₁₋₂-alkyl group, branched or linear C₁₋₈alkoxy, C₃₋₈ cycloalkyl, C₅₋₁₀ bicycloalkyl, C₆₋₁₀ tricycloalkyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenoxy, benzyl, phenethyl or phenyl propyl group, optionally substituted on their phenyl ring with 1-3 substituents Y, wherein Y has the abovementioned meaning, or R₄ represents a pyridyl or thienyl group, or R₄ represents a group NR₅R₆ wherein
R₅ and R₆ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
R₃ and R₄ - together with the nitrogen atom to which they are attached - form a saturated or unsaturated, monocyclic or bicydic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy or trifluoromethyl group or a fluoro atom,
- R₇ represents a benzyl, phenyl, thienyl or pyridyl group, which groups may be substituted on their aromatic ring with 1, 2, 3 or 4 substituents Y, wherein Y has the meaning as indicated above, which can be the same or different, or R₇ represents C₁₋₈ branched or linear alkyl, C₃₋₈ alkenyl, C₈₋₁₀ cycloalkyl, C₅₋₁₀ bicycloalkyl, C₆₋₁₀ tricycloalkyl or C₅₋₈ cycloalkenyl or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl, phenyl, hydroxy or trifluoromethyl group or a fluoro atom,
- R₈ represent a hydrogen atom or a branched or linear C₁₋₃ alkyl group,
- R₉ represents a hydrogen atom or a branched or linear C₁₋₆ alkyl, C₃₋₈ cycloalkyl or C₂₋₁₀ heteroalkyl group which groups may be substituted with a keto group, trifluoromethyl group or a fluoro atom, or R₉ represents an amino, hydroxy, phenoxy or benzyloxy group or R₉ represents a branched or linear C₁₋₈ alkoxy group, which may be substituted with a hydroxy group, a trifluoromethyl group or a fluoro atom, or R₉ represents a phenyl, benzyl, pyridyl, thienyl, pyridylmethyl or phenethyl group wherein the aromatic rings may be substituted with 1, 2 or 3 of the substituents Y, wherein Y has the meaning as indicated above, or
R₉ represents a group NR₁₀R₁₁ with the proviso that R₈ represents a hydrogen atom or a methyl group and wherein R₁₀ and R₁₁ are the same or different and represent C₁₋₄ alkyl or C₂₋₄ trifluoroalkyl or R₁₀ and R₁₁ - together with the nitrogen atom to which they are bonded - form a saturated or unsaturated heterocyclic moiety having 4 to 8 ring atoms which heterocyclic moiety contains one or two atoms from the group (O, N, S) which saturated or unsaturated heterocyclic moiety may be substituted with a C₁₋₂ alkyl group or R₈ and R₉ - together with the nitrogen atom to which they are bonded - form a saturated or unsaturated, monocyclic or bicyclic heterocyclic moiety having 4 to 10 ring atoms, which heterocyclic moiety contains one or two atoms from the group (O, N, S) or a keto group or -SO₂- group, which moiety may be substituted with a C₁₋₂ alkyl, hydroxy, phenyl, methylamino, dimethylamino, azetidinyl, pyrrolidinyl, piperidinyl or hexahydro-1H-azepinyl group,
and tautomers, stereoisomers, salts and prodrugs, which are derivatives of the compounds having formula (I) wherein a group is present which is easily removed after administration, selected from the group consisting of an amidine, enamine, a Mannich base, a hydroxyl-methylene derivative, an O-(acyloxymethylene carbamate) derivative, carbamate or enaminone.

10. Use as claimed in claim 8, **characterized in that** said disorders are eating disorders, in particular obesity, juvenile obesity and drug induced obesity.

11. Use of a compound as claimed in claim 1 or claim 2 for the preparation of a pharmaceutical composition for the treatment of eating disorders, in particular obesity, juvenile obesity and drug induced obesity, **characterized in that** said pharmaceutical composition also contains at least one lipase inhibitor.

12. Use as claimed in claim 11, **characterized in that** said lipase inhibitor is orlistat or lipstatin.

## Patentansprüche

1. Verbindungen der allgemeinen Formel (I) 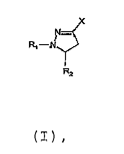 worin:
- R₁ und R₂ unabhängig Phenyl, Thienyl oder Pyridyl darstellen, welche Gruppen substituiert sein können mit 1, 2 oder 3 Substituenten Y, welche gleich oder unterschiedlich sein können, aus der Gruppe verzweigtes oder lineares C₁₋₃-Alkyl oder Alkoxy, Phenyl, Hydroxy, Chlor, Brom, Fluor, Iod, Trifluormethyl, Trifluormethylthio, Trifluormethoxy, Methylsulfonyl, Carboxyl, Trifluormethylsulfonyl, Cyano, Carbamoyl, Sulfamoyl und Acetyl, oder R₁ und/oder R₂ Naphthyl darstellen,
- X eine der Untergruppen (i) oder (ii) darstellt, 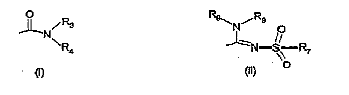
worin:
- R₃ ein Wasserstoffatom oder eine verzweigte oder lineare C₁₋₃-Alkylgruppe darstellt,
- R₄ eine verzweigte oder lineare C₁₋₈-Alkyl- oder C₃₋₈-Cycloalkyl-C₁₋₂-alkylgruppe, verzweigtes oder lineares C₁₋₈-Alkoxy, C₃₋₈-Cycloalkyl, C₅₋₁₀-Bicycloalkyl, C₆₋₁₀-Tricycloalkyl darstellt, welche Gruppen ein oder mehrere Heteroatome aus der Gruppe (O, N, S) enthalten können und welche Gruppen substituiert sein können mit einer Hydroxygruppe, 1-3 Methylgruppen, einer Ethylgruppe oder 1-3 Fluoratomen, oder R₄ eine Phenoxy-, Benzyl-, Phenethyl- oder Phenylpropylgruppe darstellt, gegebenenfalls substituiert an ihrem Phenylring mit 1-3 Substituenten Y,
worin Y die oben erwähnte Bedeutung hat, oder R₄ eine Pyridyl- oder Thienylgruppe darstellt, oder R₄ eine Gruppe NP₅R₆ darstellt, worin
R₅ und R₆ - zusammen mit dem Stickstoffatom, an welches sie gebunden sind - eine gesättigte oder ungesättigte, monocyclische oder bicyclische heterocyclische Gruppe mit 4 bis 10 Ringatomen bilden, welche heterocyclische Gruppe ein oder zwei Heteroatome aus der Gruppe (O, N , S) enthält, und welche heterocyclische Gruppe substituiert sein kann mit einer verzweigten oder linearen C₁₋₃-Alkyl-, Phenyl-, Hydroxy- oder Trifluormethylgruppe oder einem Fluoratom, oder
R₃, und R₄ - zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, und welche heterocyclische Gruppe substituiert sein kann mit einer verzweigten oder linearen C₁₋₃-Alkyl-, Phenyl-, Amino-, Hydroxy- oder Trifluormethylgruppe oder einem Fluoratom,
- R₂ eine Benzyl-, Phenyl-, Thienyl- oder Pyridylgruppe darstellt, welche Gruppen an ihrem aromatischen Ring mit 1, 2, 3 oder 4 Substituenten Y substituiert sein können, worin Y die Bedeutung hat wie oben angezeigt, welche gleich oder unterschiedlich sein können, oder R₇ für C₁₋₈ verzweigtes oder lineares Alkyl, C₃₋₈-Alkenyl, C₃₋₁₀-Cycloalkyl, C₅₋₁₀-Bicycloalkyl, C₆₋₁₀-Tricycloalkyl oder C₅₋₈-Cycloalkenyl steht oder R₇ Naphthyl darstellt oder R₇ eine Aminogruppe darstellt oder R₇ eine C₁₋₆-Dialkylaminogruppe, eine C₁₋₈-Monoalkylaminogruppe oder eine gesättigte oder ungesättigte monocyclische oder bicyclische heterocyclische Gruppe mit 4 bis 10 Ringatomen darstellt, welche heterocyclische Gruppe lader 2 Stickstoffatome enthält und welche heterocyclische Gruppe 1 Heteroatom aus der Gruppe (O, S) enthalten kann und welche heterocyclische Gruppe substituiert sein kann mit einer verzweigten oder linearen C₁₋₃-Alkyl-, Phenyl-, Hydroxy- oder Trifluormethylgruppe oder einem Fluoratom,
- R₈ ein Wasserstoffatom oder eine verzweigte oder lineare C₁₋₃-Alkylgruppe darstellt,
- R₃ ein Wasserstoffatom oder eine verzweigte oder lineare C₁₋₈-Alkyl-, C₃₋₈-Cycloalkyl- oder C₂₋₁₀Heteroalkylgruppe darstellt, welche Gruppen mit einer Ketogruppe, Trifluormethylgruppe oder einem Fluoratom substituiert sein können, oder R₉ eine Amino-, Hydroxy-, Phenoxy- oder Benzyloxygruppe darstellt, oder R₉ eine verzweigte oder lineare C₁₋₈-Alkoxygruppe darstellt, welche substituiert sein kann mit einer Hydroxygruppe, einer Trifluormethylgruppe oder einem Fluoratom, oder R₉ eine Phenyl-, Benzyl-, Pyridyl-, Thienyl-, Pyridylmethyl- oder Phenethylgruppe darstellt, worin die aromatischen Ringe mit 1, oder 3 Substituenten Y substituiert sein können, worin Y die Bedeutung wie oben angezeigt hat, oder R₉ eine Gruppe NR₁₀R₁₁ darstellt, mit der Maßgabe, dass R₉ ein Wasserstoffatom oder eine Methylgruppe darstellt und worin R₁₀ und R₁₁ gleich oder unterschiedlich sind und C₁₋₄-Alkyl oder C₂₋₄-Trifluoralkyl darstellen oder R₁₀ und R₁₁ - zusammen mit dem Stickstoffatom, an welches sie gebunden sind - eine gesättigte oder ungesättigte heterocyclische Komponente mit 4 bis 8 Ringatomen, bilden, welche heterocyclische Komponente ein oder zwei- Atome von der Gruppe (O, N, S) enthält, welche gesättigte oder ungesättigte heterocyclische Komponente mit einer C₁₋₂-Alkylgruppe substituiert sein kann, oder
R₈ und R₉ zusammen mit dem Stickstoffatom, an welches sie gebunden sind - eine gesättigte oder ungesättigte monocyclische oder bicyclische heterocyclische Komponente mit 4 bis 10 Ringatomen bilden, welche heterocyclische Komponente ein oder zwei Atome aus der Gruppe (O, N, S) enthält oder eine Ketogruppe oder -SO₂-Gruppe, welche Komponente substituiert sein kann mit einer C₁₋₂-Alkyl-, Hydroxy-, Phenyl-, Methylamino-, Dimethylamino-, Azetidinyl-, Pyrrolidinyl-, Piperidinyl- oder Hexahydro-1H-azepinylgruppe,
und alle Stereoisomere, als auch Salze und Pro-Pharmaka, welche Derivate der Verbindungen mit der Formel (I) sind, worin eine Gruppe vorhanden ist, welche nach Verabreichung leicht entfernt wird, ausgewählt aus der Gruppe bestehend aus einem Amidin, Enamin, einer Mannich-Base, einem Hydroxyl-Methylen-Derivat, einem O-(Acyloxymethylencarbamat)-Derivat, Carbamat oder Enaminon.

2. Verbindungen wie in Anspruch 1. beansprucht, der allgemeinen Formel (I) 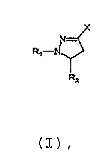 worin:
- R₁ und R₂ unabhängig Phenyl darstellen, welche Phenylgruppe substituiert sein kann mit 1, 2 oder 3 Substituenten Y, welche gleich oder unterschiedlich sein können, aus der Gruppe verzweigtes oder lineares C₁₋₃-Alkyl oder Phenyl, Hydroxy, Chlor, Brom, Fluor, Iod, Trifluormethyl, Trifluormethylthio, Trifluormethoxy, Methylsulfonyl, Carboxyl, Trifluormethylsulfonyl, Cyano, Carbamoyl, Sulfamoyl und Acetyl, oder R₁ und/oder R₂ Naphthyl, Thienyl oder Pyridyl darstellen,
- X eine der Untergruppen (i) oder (ii) darstellt, 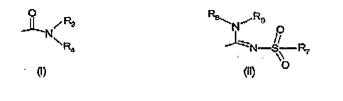
worin:
- R₃ ein Wasserstoffatom darstellt,
- R₄ eine verzweigte oder lineare C₁₋₈-Alkyl-, verzweigte oder lineare C₁₋₆-Alkoxy- oder C₃₋₈-Cycloalkylgruppe darstellt, welche Gruppen substituiert sein können mit einer Hydroxygruppe, 1-3 Methylgruppen, einer Ethylgruppe oder 1-3 Fluoratomen, oder R₄ eine Phenoxy-, Pyridyl- oder Thienylgruppe darstellt, oder R₄ eine Gruppe NR₅R₆ darstellt, worin
R₅ und R₆ - zusammen mit dem Stickstoffatom, an welches sie gebunden sind - eine gesättigte oder ungesättigte, monocyclische oder bicyclische heterocyclische Gruppe mit 4 bis 10 Ringatomen bilden, welche heterocyclische Gruppe ein oder zwei Heteroatome aus der Gruppe (O, N, S) enthält, oder
R₃ und R₄ - zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, und welche heterocyclische Gruppe substituiert sein kann mit einer Methyl-, Hydroxy- oder Trifluormethylgruppe oder einem Fluoratom,
- R₇ eine Phenylgruppe darstellt, welche Phenylgruppen an ihrem aromatischen Ring mit 1, 2, 3 oder 4 Substituenten Y substituiert sein kann, worin Y die Bedeutung hat wie oben angezeigt, welche gleich oder unterschiedlich sein können, oder R₇ für C₁₋₈ verzweigtes oder lineares Alkyl, C₃₋₁₀- Cycloalkyl oder C₅₋₁₀-Bicycloalkyl, steht oder R₇ Naphthyl darstellt oder R₇ eine Aminogruppe darstellt oder R₇ eine C₁₋₈-Dialkylaminogruppe, eine C₁₋₈-Monoalkylaminogruppe oder eine gesättigte oder ungesättigte monocyclische oder bicyclische heterocyclische Gruppe mit 4 bis 10 Ringatomen darstellt, welche heterocyclische Gruppe 1 oder 2 Stickstoffatome enthält und welche heterocyclische Gruppe 1 Heteroatom aus der Gruppe (O, S) enthalten kann und welche heterocyclische Gruppe substituiert sein kann mit einer verzweigten oder linearen C₁₋₃-Alkyl- oder Hydroxygruppe,
- R₈ ein wasserstoffatom oder eine verzweigte oder lineare C₁₋₃-Alkylgruppe darstellt,
- R₃ ein Wasserstoffatom oder eine verzweigte oder lineare C₁₋₉-Alkyl- oder C₃₋₉-Cycloalkylgruppe darstellt, welche Gruppen mit einer Trifluormethylgruppe oder einem Fluoratom substituiert sein können, oder R₉ eine Amino-, Hydroxy-, Phenoxy- oder Benzyloxygruppe darstellt, oder R₉ eine verzweigte oder lineare C₁₋₉-Alkoxygruppe darstellt, oder R₉ eine Phenylgruppe darstellt, worin der aromatische Ring mit 1, 2 oder 3 der Substituenten Y substituiert sein kann, worin Y die Bedeutung wie oben angezeigt hat, oder
R₉ eine Gruppe NR₁₀R₁₁ darstellt, mit der Maßgabe, dass R₈ ein Wasserstoffatom oder eine Methylgruppe darstellt und worin R₁₀ und R₁₁ gleich oder unterschiedlich sind und C₁₋₄-Alkyl oder C₂₋₄-Trifluoralkyl darstellen oder R₁₀ und R₁₁- zusammen mit dem Stickstoffatom, an welches sie gebunden sind - eine gesättigte oder ungesättigte heterocyclische Komponente mit 4 bis 8 Ringatomen bilden, welche heterocyclische Komponente ein oder zwei Atome aus der Gruppe (O, N, S) enthält, oder
R₈ und R₉ - zusammen mit dem Stickstoffatom, an welches sie gebunden sind - eine gesättigte oder ungesättigte monocyclische oder bicyclische heterocyclische Komponente mit 4 bis 10 Ringatomen bilden, welche heterocyclische Komponente ein oder zwei Atome aus der Gruppe (O, N, S) enthält oder eine Ketogruppe oder -SO₂-Gruppe,
und alle Stereoisomere, als auch Salze und Pro-Pharmaka, welche Derivate der Verbindungen mit der Formel (I) sind, worin eine Gruppe vorhanden ist, welche nach Verabreichung leicht entfernt wird, ausgewählt aus der Gruppe bestehend aus einem Amidin, Enamin, einer Mannich-Base, einem Hydroxyl-Methylen-Derivat, einem O-(Acyloxymethylencarbamat)-Derivat, Carbamat oder Enaminon.

3. Pharmazeutische Zusammensetzungen, umfassend zusätzlich zu einem pharmazeutisch annehmbaren Träger und/oder mindestens einer pharmazeutisch annehmbaren Hilfssubstanz eine pharmakologisch wirksame Menge von mindestens einer Verbindung nach einem der Ansprüche 1-2, oder ein Salz davon, als wirksamen Inhaltsstoff.

4. Verbindungen der allgemeinen Formel (X), Tautomere davon und Salze davon 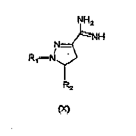 worin R₁ und R₂ die in Anspruch 1 gegebenen Bedeutungen haben, wobei solche Verbindungen in der Synthese von Verbindungen der allgemeinen Formel (I) brauchbar sind.

5. Verbindungen der Allgemeinen Formel (XI) 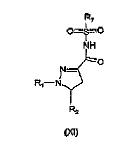 worin R₁, R₂ und R₇ die in Anspruch 1 gegebenen Bedeutungen haben, wobei solche Verbindungen in der Synthese von Verbindungen der allgemeinen Formel (I) brauchbar sind.

6. Verbindungen, der allgemeinen Formel (XII) 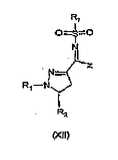 worin R₁, R₂ und R₇ die in Anspruch 1 gegebenen Bedeutungen haben und worin ein Cl- oder Br-Atom darstellt, wobei solche Verbindungen in der Synthese von Verbindungen der allgemeinen Formel (I) brauchbar sind.

7. Verbindung wie in Anspruch 1 oder Anspruch 2 beansprucht, oder ein Salz davon, zur verwendung in Medizin.

8. Verwendung einer Verbindung wie in Anspruch 1 oder Anspruch 2 beansprucht für die Herstellung einer pharmazeutischen Zusammensetzung für die Behandlung von Störungen, welche Cannabinoid-Neurotransmission einbeziehen, wie Psychose, Angst, Depression, Aufmerksamkeitsdefiziten, Gedächtnisstörungen, kognitiven Störungen, Appetitstörungen, Fettsucht, insbesondere juveniler Fettsucht und Arznei- bzw. Drogen-induzierter. Fettsucht, Sucht, Impulskontrollstörungen, Appetenz, Arznei- bzw. Drogenabhängigkeit und neurologischen Störungen wie neurodegenerativen Störungen, Demenz, Dystonie, Muskelspastizität, Tremor, Multiple Sklerose, traumatischer Hirnverletzung, Parkinson-Krankheit, Alzheimer-Krankheit, Epilepsie, Huntington-Krankheit, Tourette-Syndrom, zerebraler Ischämie, zerebraler Apoplexie, kraniozerebralem Trauma, Schlaganfall, Rückenmarksverletzung, neuroentzündlichen Störungen, Plaque-Sklerose, viraler Enzephalitis, demyelinisierungsbezogenen Störungen, als auch für die Behandlung von. Schmerzstörungen, einschließlich neuropathischen Schmerzstörungen, und anderen Krankheiten, welche Cannabinoid-Neurotransmission einbeziehen, einschließlich der Behandlung von septischem Schock, Glaukom, Krebs, Diabetes, Erbrechen, Übelkeit, Asthma, Atemwegserkrankungen, gastrointestinalen Störungen, Magengeschwüren, Diarrhoe, kardiovaskulären Störungen, Atherosklerose, Leberzirrhose und sexuellen Störungen.

9. Verfahren zum Herstellen einer pharmazeutischen Zusammensetzung, **dadurch gekennzeichnet, dass** eine Verbindung der Formel (I) verwendet wird 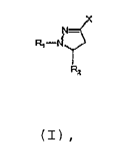 worin:
- R₁ und R₂ unabhängig Phenyl, Thienyl oder Pyridyl darstellen, welche Gruppen substituiert sein können mit 1, 2 oder 3 Substituenten Y, welche gleich oder unterschiedlich sein können, aus der Gruppe C₁₋₃-Alkyl oder Alkoxy, Phenyl, Hydroxy, Chlor, Brom, Fluor, Iod, Trifluormethyl, Trifluormethylthio, Trifluormethoxy, Methylsulfonyl, Carboxyl, Trifluormethylsulfonyl, Cyano, Carbamoyl, Sulfamoyl und Acetyl, oder R₁ und/oder R₂ Naphthyl darstellen,
- X eine der Untergruppen (i) oder (ii) darstellt, 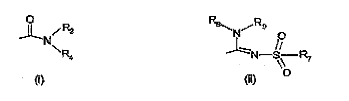
worin:
- R₃ ein Wasserstoffatom oder eine verzweigte oder lineare C₁₋₃-Alkylgruppe darstellt,
- R₄ eine verzweigte oder lineare C₁₋₆-Alkyl- oder C₃₋₈-Cycloalkyl-C₁₋₂₋Alklgruppe, verzweigtes oder lineares C₁₋₆-Alkoxy, C₃₋₈-Cycloalkyl, C₅₋₁₀-Bicycloalkyl, C₆₋₁₀-Tricycloalkyl darstellt, welche Gruppen ein oder mehrere Heteroatome aus der Gruppe (O, N, S) enthalten können und welche Gruppen substituiert sein können mit einer Hydroxygruppe, 1-3 Methylgruppen, einer Ethylgruppe oder 1-3 Fluoratomen, oder R₄ eine Phenyl-, Phenoxy-, Benzyl-, Phenethyl- oder Phenylpropylgruppe darstellt, gegebenenfalls substituiert an ihrem Phenylring mit 1-3 Substituenten Y, worin Y die oben erwähnte Bedeutung hat, oder R₄ eine Pyridyl- oder Thienylgruppe darstellt, oder R₄ eine Gruppe NR₅R₆ darstellt, worin R₅ und R₆ - zusammen mit dem Stickstoffatom, an welches sie gebunden, sind eine gesättigte oder ungesättigte, monocyclische oder bicyclische heterocyclische Gruppe mit 4 bis 10 Ringatomen bilden, welche heterocyclische Gruppe ein oder mehr Heteroatome aus den Gruppen (O, N, S) enthält, und welche heterocyclische Gruppe substituiert sein kann mit einer verzweigten oder linearen C₁₋₃-Alkyl-, Phenyl-, Hydroxy- oder Trifluormethylgruppe oder einem Fluoratom, oder R₃ und R₄
- zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder mehrere Heteroatome aus der Gruppe (O, N, S) enthält, und welche heterocyclische Gruppe substituiert sein kann mit einer verzweigten oder linearen C₁₋₃-Alkyl-, Phenyl-, Amino-, Hydroxy- oder Trifluormethylgruppe oder einem Fluoratom, oder
- R₇ eine Benzyl-, Phenyl-, Thienyl- oder Pyridylgruppe darstellt, welche Gruppen an ihrem aromatischen Ring mit 1, 2, 3 oder 4 Substituenten Y substituiert sein können, worin Y die Bedeutung hat wie oben angezeigt, welche gleich oder unterschiedlich sein können, oder R₇ für C₁₋₈, verzweigtes oder lineares Alkyl, C₃₋₈-Alkenyl, C₃₋₁₀-Cycloalkyl, C₅₋₁₀-Bicycloalkyl, C₆₋₁₀-Tricycloalkyl oder C₅₋₈-Cycloalkenyl steht oder R₇ Naphthyl darstellt oder R₇ eine Aminogruppe darstellt oder R₇ eine C₁₋₈-Dialkylaminogruppe, eine C₁₋₈-Monoalkylaminogruppe oder eine gesättigte oder ungesättigte monocyclische oder bicyclische heterocyclische Gruppe mit 4 bis 10 Ringatomen darstellt, welche heterocyclische Gruppe 1 oder 2 Stickstoffatome enthält und welche heterocyclische Gruppe 1 Heteroatom aus der Gruppe (O, S) enthalten kann und welche heterocyclische Gruppe substituiert sein kann mit einer verzweigten oder linearen C₁₋₃-Alkyl-, Phenyl-, Hydroxy- oder Trifluormethylgruppe oder einem Fluoratom,
- R₈ ein Wasserstoffatom oder eine verzweigte oder lineare C₁₋₃-Alkylgruppe darstellt,
- R₉ ein Wasserstoffatom oder eine verzweigte oder lineare C₁₋₈-Alkyl-, C₃₋₈-cycloalkyl- oder C₂₋₁₀-Heteroalkylgruppe darstellt, welche Gruppen mit einer Ketogruppe, Trifluormethylgruppe oder einem Fluoratom substituiert sein können, oder R₉ eine Amino-, Hydroxy-, Phenoxy- oder Benzyloxygruppe darstellt, oder R₉ eine verzweigte oder lineare C₁₋₈-Alkoxygruppe darstellt, welche substituiert sein kann mit einer Hydroxygruppe, einer Trifluormethylgruppe oder einem Fluoratom, oder R₉ eine Phenyl-, Benzyl-, Pyridyl-, Thienyl-, Pyridylmethyl- oder Phenethylgruppe darstellt, worin die aromatischen Ringe mit 1, 2 oder 3 der Substituenten Y substituiert sein können, worin Y die Bedeutung wie oben angezeigt hat, oder
R₉ eine Gruppe NR₁₀R₁₁ darstellt, mit der Maßgabe, dass R₈ ein Wasserstoffatom oder eine Methylgruppe darstellt und worin R₁₀ und R₁₁ gleich oder unterschiedlich sind und C₁₋₄-Alkyl oder C₂₋₄-Trifluoralkyl darstellen oder R₁₀ und R₁₁ - zusammen mit dem Stickstoffatom, an welches sie gebunden sind eine gesättigte oder ungesättigte heterocyclische Komponente mit 4 bis 8 Ringatomen, bilden, welche heterocyclische Komponente ein oder zwei Atome aus der Gruppe (O, N, S) enthält, welche gesättigte oder ungesättigte heterocyclische Komponente mit einer C₁₋₂-Alkylgruppe substituiert sein kann, oder
R₈ und R₉ - zusammen mit dem Stickstoffatom, an welches sie gebunden sind - eine gesättigte oder ungesättigte monocyclische oder bicyclische heterocyclische Komponente mit 4 bis 10 Ringatomen bilden, welche heterocyclische Komponente ein oder zwei Atome aus der Gruppe (O, N, S) enthält oder eine Ketogruppe oder -SO₂-Gruppe, welche Komponente substituiert sein kann mit einer C₁₋₂-Alkyl-, Hydroxy-, Phenyl-, Methylamino-, Dimethylamino-, Azetidinyl-, Pyrrolidinyl-, Piperidinyl- oder Hexahydro-1H-azepinylgruppe,
und Tautomere, Stereoisomere, Salze und Pro-Pharmaka, welche Derivate der Verbindungen mit der Formel (I) sind, worin eine Gruppe vorhanden ist, welche nach Verabreichung leicht entfernt wird, ausgewählt aus der Gruppe bestehend aus einem Amidin, Enamin, einer Mannich-Base, einem Hydroxyl-Methylen-Derivat, einem O-(Acyloxymethylencarbamat)-Derivat, Carbamat oder Enaminon.

10. Verwendung wie in. Anspruch 8 beansprucht, **dadurch gekennzeichnet, dass** besagte Störungen Essstörungen sind, insbesondere Fettsucht, juvenile Fettsucht und Arznei- bzw. Drogen-induzierte Fettsucht.

11. Verwendung einer Verbindung wie in Anspruch 1 oder. Anspruch 2 beansprucht für die Herstellung einer pharmazeutischen Zusammensetzung für die Behandlung von Essstörungen, insbesondere Fettsucht, juveniler Fettsucht und Arznei- bzw. Drogen-induzierter Fettsucht, **dadurch gekennzeichnet, dass** besagte pharmazeutische Zusammensetzung ebenfalls mindestens einen Lipasehemmer enthält.

12. Verwendung wie in Anspruch 11 beansprucht, **dadurch gekennzeichnet, dass** besagter Lipasehemmer Orlistat oder Lipstatin ist.

## Revendications

1. Composés de la formule générale (I) 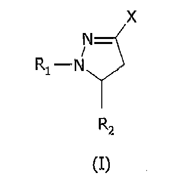 dans laquelle :
- R₁ et R₂ représentent indépendamment des groupes phényle, thiényle ou pyridyle, lesquels groupes peuvent être substitués avec 1, 2 ou 3 substituants Y, lesquels peuvent être identiques ou différents, choisis parmi des groupes alkyle ou alcoxy en C₁-C₃ linéaire ou ramifié, phényle, hydroxy, chloro, bromo, fluoro, iodo, trifluorométhyle, trifiluorométhylthio, trifluoro-méthoxy, méthylsulfonyle, carboxyle, trifluorométhylsulfonyle, cyano, carbamoyle, sulfamoyle et acétyle, ou R₁ et/ou R₂ représentent le groupe naphtyle,
- X représente un des sous-groupes (i) ou (ii), 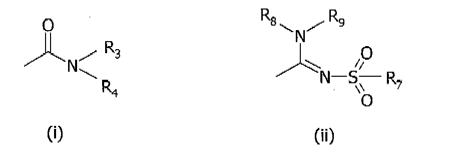
dans lesquelles
- R₃ représente un atome d'hydrogène ou un groupe alkyle en C₁-C₃ linéaire ou ramifié,
- R₄ représente un groupe alkyle en C₁-C₈ linéaire ou ramifié ou (cycloalkyle en C₃-C₈)-(alkyle en C₁-C₂), un groupe alcoxy en C₁-C₈ linéaire ou ramifié, cycloalkyle en C₃-C₈, bicycloalkyle en C₅-C₁₀, tricycloalkyle en C₆-C₁₀, lesquels groupes peuvent contenir un ou plusieurs hétéroatomes choisis parmi O, N, S et lesquels groupes peuvent être substitués avec un groupe hydroxy, 1-3 groupes méthyle, un groupe éthyle ou 1-3 atomes de fluor, ou R₄ représente un groupe phénoxy, benzyle, phénéthyle ou phénylpropyle facultativement substitué sur leur cycle phényle avec 1-3 substituants Y, où Y a la signification citée ci-dessus, ou R₄ représente un groupe pyridyle ou thiényle, ou R₄ représente un groupe NR₅R₆ dans lequel
R₅ et R₆ - avec l'atome d'azote auquel ils sont fixés - forment un groupe hétérocyclique, monocyclique ou bicyclique, saturé ou insaturé ayant de 4 à 10 atomes de cycle, lequel groupe hétérocyclique contient un ou deux hétéroatomes choisis parmi O, N, S et lequel groupe hétérocyclique peut être substitué avec un groupe alkyle en C₁-C₃ linéaire ou ramifié, phényle, hydroxy ou trifluorométhyle ou un atome de fluor, ou
R₃ et R₄ - avec l'atome d'azote auquel ils sont fixés - forment un groupe hétérocyclique, monocyclique ou bicyclique, saturé ou insaturé ayant de 4 à 10 atomes au noyau, lequel groupe hétérocyclique contient un ou deux hétéroatomes choisis parmi O, N, S at lequel groupe hétérocyclique peut être substitué avec un groupe alkyle en C₁-C₃ linéaire ou ramifié, phényle, amino, hydroxy ou trifluorométhyle ou un atome de fluor,
R₇ représente un groupe benzyle, phényle, thiényle ou pyridyle, lesquels groupes peuvent être substitués sur leur cycle aromatique avec 1, 2, 3 ou 4 substituants Y, où Y a la signification indiquée ci-dessus, lesquels peuvent être identiques ou différents, ou R₇ représente un groupe alkyle en C₁-C₈ linéaire ou ramifié, alcényle en C₃-C₈, cycloalkyle en C₃-C₁₀, bicycloalkyle en C₅-C₁₀, tricycloalkyle en C₆-C₁₀ ou cycloalcényle en C₆-C₈ ou R₇ représente le groupe naphtyle ou R₇ représente un groupe amino ou R₇ représente un groupe dialkylamino en C₁-C₈, un groupe monoalkylamino en C₁-C₈ ou un groupe hétérocyclique, monocyclique ou bicyclique, saturé ou insaturé ayant de 4 à 10 atomes au noyau, lequel groupe hétérocyclique contient 1 ou 2 atomes d'azote et lequel groupe hétérocyclique peut contenir hétéroatome choisi parmi 0, S et lequel groupe hétérocyclique peut être substitué avec un groupe alkyle en C₁-C₃ linéaire ou ramifié, phényle, hydroxy ou trifluorométhyle ou un atome de fluor,
- R₈ représente un atome d'hydrogène ou un groupe alkyle en C₁-C₃ linéaire ou ramifié,
- R₉ représente un atome d'hydrogène ou un groupe alkyle en C₁-C₈ linéaire ou ramifié, cycloalkyle en C₃-C₈ ou hétéroalkyle en C₂-C₁₀, lesquels groupes peuvent être substitués avec un groupe céto, un groupe trifluorométhyle ou un atome de fluor, ou R₉ représente un groupe amino, hydroxy, phénoxy ou benzyloxy ou R₉ représente un groupe alcoxy en C₁-C₈ linéaire ou ramifié qui peut être substitué avec un groupe hydroxy, un groupe trifluorométhyle ou un atome de fluor, ou R₉ représente un groupe phényle, benzyle, pyridyle, thiényle, pyridylméthyle ou phénéthyle, dans lequel les cycles aromatiques peuvent être substitués avec 1, 2 ou 3 des substituants Y, où Y a la signification indiquée ci-dessus, ou
R₉ représente un groupe NR₁₀R₁₁ à condition que R₈ représente un atome d'hydrogène ou un groupe méthyle et où R₁₀ et R₁₁ sont identiques ou différents et représentent un groupe alkyle en C₁-C₄ ou trifluoroalkyle en C₂-C₄ ou R₁₀ et R₁₁ - avec l'atome d'azote auquel ils sont fixés - forment un fragment hétérocyclique saturé ou insaturé ayant de 4 à 8 atomes de cycle, lequel fragment hétérocyclique contient un ou deux atomes choisis parmi O, N, S, lequel fragment hétérocyclique saturé ou insaturé peut être substitué avec un groupe alkyle en C₁-C₂ ou
R₈ et R₉ avec l'atome d'azote auquel ils sont fixés - forment un fragment hétérocyclique, monocyclique ou bicyclique, saturé ou insaturé ayant de 4 à 10 atomes au noyau, lequel fragment hétérocyclique contient un ou deux atomes choisis parmi O, N, S ou un groupe céto ou un groupe -SO₂-, lequel fragment peut être substitué avec un groupe alkyle en C₁-C₂, hydroxy, phényle, méthylamino, diméthylamino, azétidinyle, pyrrolidinyle, pipéridinyle ou hexahydro-1H-azépinyle,
et tous les stéréoisomères ainsi que sels et promédicaments qui sont des dérivés des composés présentant la formule (I), dans laquelle un groupe est présent, lequel peut être facilement éliminé après l'administration, choisi parmi une amidine, une énamine, une base de Mannich, un dérivé d'hydroxyl-méthylène, un dérivé d'O-(carbamate d'acyloxyméthylène), un carbamate ou une énaminone.

2. Composés selon la revendication 1 de la formule générale (I) 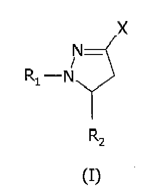 dans laquelle :
- R₁ et R₂ représentent indépendamment un groupe phényle, lequel groupe phényle peut être substitué avec 1, 2 ou 3 substituants Y, lesquels peuvent être identiques ou différents, choisis parmi un groupe alkyle ou alcoxy en C₁-C₃ linéaire ou ramifié, phényle, hydroxy, chloro, bromo, fluoro, iodo, trifluorométhyle, trifluorométhylthio, trifluoro-méthoxy, méthylsulfonyle, carboxyle, trifluorométhylsulfonyle, cyano, carbamoyle, sulfamoyle et acétyle, ou R₁ et/ou R₂ représentent le groupe naphtyle, thiényle ou pyridyle,
- X représente un des sous-groupes (i) ou (ii), 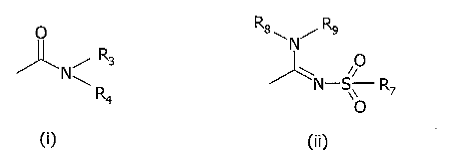
dans lesquelles
- R₃ représente un atome d'hydrogène,
- R₄ représente un groupe alkyle en C₁-C₈ linéaire ou ramifié alcoxy en C₁-C₈ linéaire ou ramifié ou cycloalkyle en C₃-C₈, lesquels groupes peuvent être substitués avec un groupe hydroxy, 1-3 groupes méthyle, un groupe éthyle ou 1-3 atomes de fluor, ou R₄ représente un groupe phénoxy, pyridyle ou thiényle, ou R₄ représente un groupe NR₃R₆ dans lequel
R₅ et R₆ - avec l'atome d'azote auquel ils sont fixés - forment un groupe hétérocyclique, monocyclique ou bicyclique, saturé ou insaturé ayant de 4 à 10 atomes au noyau, lequel groupe hétérocyclique contient un ou deux hétéroatomes choisis parmi O, N, S ou
R₃ et R₄- avec l'atome d'azote auquel ils sont fixés - forment un groupe hétérocyclique, monocyclique ou bicyclique, saturé ou insaturé ayant de 4 à 10 atomes au noyau, lequel groupe hétérocyclique contient un ou deux hétéroatomes choisis parmi O, N, S et lequel groupe hétérocyclique peut être substitué avec un groupe, méthyle, hydroxy ou trifluorométhyle ou un atome de fluor,
- R₇ représente un groupe phényle, lequel groupe phényle peut être substitué sur son cycle aromatique avec 1, 2, 3 ou 4 substituants Y, où Y a la signification indiquée ci-dessus, lesquels peuvent être identiques ou différents, ou R₇ représente un groupe alkyle en C₁-C₈ linéaire ou ramifié, cycloalkyle en C₃-C₁₀, bicycloalkyle en C₅-C₁₀, ou R₇ représente le groupe naphtyle ou R₇ représente un groupe amino ou R₇ représente un groupe dialkylamino en C₁-C₈, un groupe monoalkyl-amino en C₁-C₈ ou un groupe hétérocyclique, monocyclique ou bicyclique, saturé ou insaturé ayant de 4 à 10 atomes au noyau, lequel groupe hétérocyclique contient 1 ou 2 atomes d'azote et lequel groupe hétérocyclique peut contenir 1 hétéroatome choisi parmi O, 5 et lequel groupe hétérocyclique peut être substitué avec un groupe alkyle en C₁-C₃ linéaire ou ramifié, ou hydroxy,
- R₈ représente un atome d'hydrogène ou un groupe alkyle en C₁-C₃ linéaire ou ramifié,
- R₉ représente un atome d'hydrogène ou un groupe alkyle en C₁-C₈ linéaire ou ramifié ou un groupe cycloalkyle en C₃-C₈, lesquels groupes peuvent être substitués avec un groupe trifluorométhyle ou un atome de fluor, ou R₉ représente un groupe amino, hydroxy, phénoxy ou benzyloxy ou R₉ représente un groupe alcoxy en C₁-C₈ linéaire ou ramifié, ou R₉ représente un groupe phényle, dans lequel le cycle aromatique peut être substitué avec 1, 2 ou 3 des substituants Y, où Y a la signification indiquée ci-dessus, ou
R₉ représente un groupe NR₁₀R₁₁ à condition que R₈ représente un atome d'hydrogène ou un groupe méthyle et où R₁₀ et R₁₁ sont identiques ou différents et représentent un groupe alkyle en C₁-C₄ ou trifluoroalkyle en C₂-C₄ ou R₁₀ et R₁₁ - avec l'atome d'azote auquel ils sont fixés - forment un fragment hétérocyclique saturé ou insaturé ayant de 4 à 8 atomes au noyau, lequel fragment hétérocyclique contient un ou deux atomes choisis parmi O, N, S ou
R₈ et R₉ - avec l'atome d'azote auquel ils sont fixés - forment un fragment hétérocyclique, monocyclique ou bicyclique, saturé ou insaturé ayant de 4 à 10 atomes au noyau, lequel fragment hétérocyclique contient un ou deux atomes choisis parmi O, N, S ou un groupe céto ou un groupe -SO₂-, et tous les stéréoisomères ainsi que sels et promédicaments qui sont des dérivés des composés présentant la formule (I) dans laquelle un groupe est présent, lequel peut être facilement éliminé après l'administration, choisi parmi une amidine, une énamine, une base de Mannich, un dérivé d'hydroxyl-méthylène, un dérivé d'O-(carbamate d'acyloxyméthylène), un carbamate ou une énaminone.

3. Compositions pharmaceutiques comprenant, en plus d'un support pharmaceutiquement acceptable et/ou d'au moins une substance auxiliaire pharmaceutiquement acceptable, une quantité pharmacologiquement active d'au moins un composé d'une des revendications 1-2 ou d'un sel de celui-ci comme ingrédient actif.

4. Composés de la formule générale (X), tautomères de ceux-ci et sels de ceux-ci 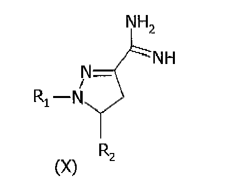 dans laquelle R₁ et R₂ ont les significations données dans la revendication 1, de tels composés étant utiles dans la synthèse de composés de la formule générale (I).

5. Composés de la formule générale (XI) 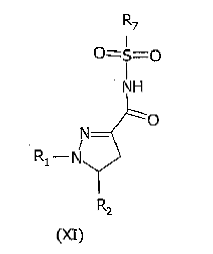 dans laquelle R₁, R₂ et R₇ ont les significations données dans la revendication 1, de tels composés étant utiles dans la synthèse de composés de la formule générale (I).

6. Composés de la formule générale (XII) 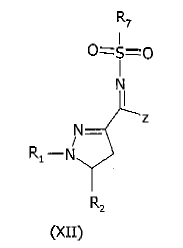 dans laquelle R₁, R₂ et R₇ ont les significations données dans la revendication 1 et dans laquelle Z représente un atome de Cl ou de Br, de tels composés étant utiles dans la synthèse de composés de la formule générale (1).

7. Composé selon la revendication 1 ou la revendication 2 ou sel de celui-ci pour une utilisation en médecine.

8. Utilisation d'un composé selon la revendication 1 ou la revendication 2 pour la préparation d'une composition pharmaceutique destinée au traitement de troubles impliquant la neurotransmission de cannabinoïdes, tels que la psychose, l'anxiété, la dépression, les déficits de l'attention, les troubles de la mémoire, les troubles cognitifs, les troubles de l'appétit, l'obésité, en particulier l'obésité juvénile et l'obésité induite par les médicaments, l'addiction, les troubles du contrôle des pulsions, l'appétence, la dépendance aux médicaments et les troubles neurologiques, tels que les troubles neurodégénératifs, la démence, la dystonie, la spasticité du muscle, les tremblements, la sclérose en plaques, la lésion cérébrale traumatique, la maladie de Parkinson, la maladie d'Alzheimer, l'épilepsie, la maladie de Huntington, le syndrome de Tourette, l'ischémie cérébrale, l'apoplexie cérébrale, les traumatismes crâniocérébraux, l'attaque, la lésion de la moelle épinière, les troubles neuroinflammatoires, la sclérose en plaques, l'encéphalite virale, les troubles liés à la démyélinisation ainsi que pour le traitement des troubles de la douleur comprenant les troubles de la douleur neuropathique et d'autres maladies impliquant la neurotransmission de cannabinoïdes incluant le traitement du choc septique, du glaucome, du cancer, des diabètes, du vomissement, des nausées, des asthmes, des maladies respiratoires, des troubles gastro-intestinaux, des ulcères gastriques, des diarrhées, des troubles cardio-vasculaires, de l'athérosclérose, de la cirrhose du foie et des troubles sexuels.

9. Procédé de préparation d'une composition pharmaceutique **caractérisé en ce que** l'on utilise un composé de la formule (I) 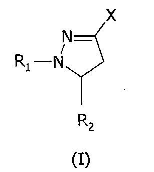 dans laquelle :
- R₁ et R₂ représentent indépendamment les groupes phényle, thiényle ou pyridyle, lesquels groupes peuvent être substitués avec 1, 2 ou 3 substituants Y, qui peuvent être identiques ou différents, choisis parmi un groupe alkyle ou alcoxy en C₁-C₃ linéaire ou ramifié, phényle, hydroxy, chloro, bromo, fluoro, iodo, trifluorométhyle, trifluorométhylthio, trifluoro-méthoxy, méthylsulfonyle, carboxyle, trifluorométhylsulfonyle, cyano, carbamoyle, sulfamoyle et acétyle, ou R₁ et/ou R₂ représentent le groupe naphtyle,
- X représente un des sous-groupes (i) ou (ii), 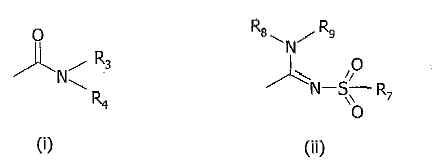
dans lesquelles
- R₃ représente un atome d'hydrogène ou un groupe alkyle en C₁-C₃ linéaire ou ramifié,
- R₄ représente un groupe alkyle en C₁-C₈ linéaire ou ramifié ou (cycloalkyle en C₃-C₈)-(alkyle en C₁-C₂), alcoxy en C₁-C₈ linéaire ou ramifié, cycloalkyle en C₃-C₈, bicycloalkyle en C₅-C₁₀, tricycloalkyle en C₆-C₁₀, lesquels groupes peuvent contenir un ou plusieurs hétéroatomes choisis parmi O, N, S et lesquels groupes peuvent être substitués avec un groupe hydroxy, 1-3 groupes méthyle, un groupe éthyle ou 1-3 atomes de fluor, ou R₄ représente un groupe phényle, phénoxy, benzyle, phénéthyle ou phénylpropyle facultativement substitué sur leur noyau phényle avec 1-3 substituants Y, où Y a la signification citée ci-dessus, ou R₄ représente un groupe pyridyle ou thiényle, ou R₄ représente un groupe NR₅R₆ dans lequel
- R₅ et R₆ - avec l'atome d'azote auquel ils sont fixés - forment un groupe hétérocyclique, monocyclique ou bicyclique, saturé ou Insaturé ayant de 4 à 10 atomes au noyau, lequel groupe hétérocyclique contient un ou deux hétéroatomes choisis parmi O, N, S et lequel groupe hétérocyclique peut être substitué avec un groupe alkyle en C₁-C₃ linéaire ou ramifié, phényle, hydroxy ou trifluorométhyle ou un atome de fluor, ou
- R₃ et R₄ - avec l'atome d'azote auquel ils sont fixés - forment un groupe hétérocyclique, monocyclique ou bicyclique, saturé ou insaturé ayant de 4 à 10 atomes au noyau, lequel groupe hétérocyclique contient un ou deux hétéroatomes choisis parmi O, N, S et lequel groupe hétérocyclique peut être substitué avec un groupe alkyle en C₁-C₃ linéaire ou ramifié, phényle, amino, hydroxy ou trifluorométhyle ou un atome de fluor,
R₇ représente un groupe benzyle, phényle, thiényle ou pyridyle, lesquels groupes peuvent être substitués sur leur cycle aromatique avec 1, 2, 3 ou 4 substituants Y, où Y a la signification indiquée ci-dessus, lesquels peuvent être identiques ou différents, ou R₇ représente un groupe alkyle en C₁-C₈ linéaire ou ramifié, alcényle en C₃-C₈, cycloalkyle en C₃-C₁₀, bicycloalkyle en C₅-C₁₀, tricycloalkyle en C₆-C₁₀ ou cycloalcényle en C₅-C₈ ou R₇ représente le groupe naphtyle ou R₇ représente un groupe amino ou R₇ représente un groupe dialkylamino en C₁-C₈, un groupe monoalkylamino en C₁-C₈ ou un groupe hétérocyclique, monocyclique ou bicyclique, saturé ou insaturé ayant de 4 à 10 atomes au noyau, lequel groupe hétérocyclique contient 1 ou 2 atomes d'azote et lequel groupe hétérocyclique peut contenir 1 hétéroatome choisi parmi 0, S et lequel groupe hétérocyclique peut être substitué avec un groupe alkyle en C₁-C₃ linéaire ou ramifié, phényle, hydroxy ou trifluorométhyle ou un atome de fluor,
- R₈ représente un atome d'hydrogène ou un groupe alkyle en C₁-C₃ linéaire ou ramifié,
- R₉ représente un atome d'hydrogène ou un groupe alkyle en C₁-C₈ linéaire ou ramifié, cycloalkyle en C₃-C₈ ou hétéroalkyle en C₂-C₁₀, lesquels groupes peuvent être substitués avec un groupe céto, un groupe trifluorométhyle ou un atome de fluor, ou R₉ représente un groupe amino, hydroxy, phénoxy ou benzyloxy ou R₉ représente un groupe alcoxy en C₁-C₈ linéaire ou ramifié qui peut être substitué avec un groupe hydroxy, un groupe trifluorométhyle ou un atome de fluor, ou R₉ représente un groupe phényle, benzyle, pyridyle, thiényle, pyridylméthyle ou phénéthyle dans lequel les cycles aromatiques peuvent être substitués avec 1, 2 ou 3 des substituants Y, où Y a la signification indiquée ci-dessus, ou
R₉ représente un groupe NR₁₀R₁₁ à condition que R₈ représente un atome d'hydrogène ou un groupe méthyle et où R₁₀ et R₁₁ sont identiques ou différents et représentent un groupe alkyle en C₁-C₄ ou trifluoroalkyle en C₂-C₄ ou R₁₀ et R₁₁ - avec l'atome d'azote auquel ils sont fixés- forment un fragment hétérocyclique saturé ou insaturé ayant de 4 à 8 atomes au noyau, lequel fragment hétérocyclique contient un ou deux atomes choisis parmi O, N, S, lequel fragment hétérocyclique saturé ou insaturé peut être substitué avec un groupe alkyle en C₁-C₂ ou
R₈ et R₉ - avec l'atome d'azote auquel ils sont fixés- forment un fragment hétérocyclique, monocyclique ou bicyclique, saturé ou insaturé ayant de 4 à 10 atomes de cycle, lequel fragment hétérocyclique contient un ou deux atomes choisis parmi O, N, S ou un groupe céto ou un groupe -SO₂-, lequel fragment peut être substitué avec un groupe alkyle en C₁-C₂, hydroxy, phényle, méthylamino, diméthylamino, azétidinyle, pyrrolidinyle, pipéridinyle ou hexahydro-1H-azépinyle,
et les tautomères, les stéréoisomères ainsi que les sels et les promédicaments qui sont des dérivés des composés présentant la formule (I), dans laquelle un groupe est présent, lequel peut être facilement éliminé après l'administration, choisi parmi une amidine, une énamine, une base de Mannich, un dérivé d'hydroxyl-méthylène, un dérivé d'O-(carbamate d'acyloxyméthyléne), un carbamate ou une énaminone.

10. Utilisation selon la revendication 8, **caractérisée en ce que** lesdits troubles sont des troubles alimentaires, en particulier l'obésité, l'obésité juvénile et l'obésité induite par les médicaments.

11. Utilisation d'un composé selon la revendication 1 ou la revendication 2 pour la préparation d'une composition pharmaceutique destinée au traitement des troubles alimentaires, en particulier de l'obésité, de l'obésité juvénile et de l'obésité induite par des médicaments, **caractérisée en ce que** ladite composition pharmaceutique contient également au moins un inhibiteur de lipase.

12. Utilisation selon la revendication 11, **caractérisée en ce que** ledit inhibiteur de lipase est l'orlistat ou la lipstatine.
